# EUROPEAN PATENT SPECIFICATION

(11) **EP 0 981 537 B1**
(45) Date of publication and mention of the grant of the patent: **18.07.2007**
(21) Application number: 98922118.9
(22) Date of filing: 06.05.1998
(51) Int. Cl.: C07H 21/04, C12P 19/34, C12N 5/06, C12N 5/08

(54) **AN INFECTIOUS CLONE FOR HUMAN PARAINFLUENZA VIRUS TYPE 3**
INFEKTIÖSER KLON FÜR MENSCHLICHEN PARAINFLUENZAVIRUS-TYP
CLONE INFECTIEUX ASSOCIE AU VIRUS PARAINFLUENZA DE TYPE 3 D'ORIGINE HUMAINE

(30) Priority: 07.05.1997 US 45805
(43) Date of publication of application: 01.03.2000
(73) Proprietor: THE CLEVELAND CLINIC FOUNDATION, Cleveland, OH 44195 (US)
(72) Inventor: BANERJEE, Amiya, K., Moreland Hills, OH 44022 (US); HOFFMAN, Michael, A., Shaker Heights, OH 44122 (US)
(74) Representative: Walters, Philip Bernard William
(86) International application number: PCT/US1998/009270
(87) International publication number: WO 1998/050405

(56) References cited:
- EP-A- 0 702 085
- HOFFMAN MICHAEL A ET AL: "An infectious clone of human parainfluenza virus type 3." JOURNAL OF VIROLOGY, vol. 71, no. 6, 1997, pages 4272-4277, XP001088561 ISSN: 0022-538X
- COLLINS P L ET AL: "PRODUCTION OF INFECTIOUS HUMAN RESPIRATORY SYNCYTIAL VIRUS FROM CLONED CDNA CONFIRMS AN ESSENTIAL ROLE FOR THE TRANSCRIPTION ELONGATION FACTOR FROM THE 5' PROXIMAL OPEN READING FRAME OF THE M2 MRNA IN GENE EXPRESSION AND PROVIDES A CAPABILITY FOR VACCINE DEVELOPMENT" PROCEEDINGS OF THE NATIONAL ACADEMY OF SCIENCES OF USA, NATIONAL ACADEMY OF SCIENCE. WASHINGTON, US, vol. 92, 1 December 1995 (1995-12-01), pages 11563-11567, XP002066592 ISSN: 0027-8424
- YU QINGZHONG ET AL: "Functional cDNA clones of the human respiratory syncytial (RS) virus N, P and L proteins support replication of RS virus genomic RNA analogs and define minimal trans-acting requirements for RNA replication." JOURNAL OF VIROLOGY, vol. 69, no. 4, 1995, pages 2412-2419, XP001088562 ISSN: 0022-538X
- DE BISHNU P ET AL: "Rescue of synthetic analogs of genome RNA of human parainfluenza virus type 3." VIROLOGY, vol. 196, no. 1, 1993, pages 344-348, XP001084270 ISSN: 0042-6822
- SIDHU MOHINDERJIT S ET AL: "Rescue of synthetic measles virus minireplicons: Measles genomic termini direct efficient expression and propagation of a reporter gene." VIROLOGY, vol. 208, no. 2, 1995, pages 800-807, XP002022942 ISSN: 0042-6822
- GARCIN et al., "A Highly Recombinogenic System for the Recovery of Infectious Sendai Paramyxovirus from cDNA: Generation of a Novel Copy-Back Nondefective Interfering Virus", THE EMBO JOURNAL, 1995, Volume 14, No. 24, pages 6087-6094, XP002911573
- KATO et al., "Initiation of Sendai Virus Multiplication from Transfected cDNA or RNA with Negative or Positive Sense", GENES TO CELLS, June 1996, Volume 1, pages 569-579, XP002911572

## Description

This work was supported in part by National Institutes of Health Grant No. 32027 from the Institute of Allergy and Infectious Diseases. The government may have rights in this invention.

### BACKGROUND

Recognized in 1956 as a cause of respiratory infection in man, human parainfluenza viruses (HPIV) are believed to account for 4 to 22 percent of the respiratory illnesses in children, second only to respiratory syncytial virus in this regard. HPIV are important causes of the lower respiratory tract diseases such as pneumonia and bronchiolitis, and are the most common cause of croup in young children. Of the four HPIV serotypes, 1-4, type 3 virus (HPIV-3), appears to be the most virulent, frequently causing bronchiolitis and pneumonia during the first month of life.

Unfortunately, effective vaccines or antiviral therapies, which can be used to prevent or treat HPIV-induced infections, are not presently available. Standard methods which are used to produce inactive viruses, such as heat inactivation or chemical treatment of the virus, have been unsuccessful with all HPIV strains and serotypes, including HPIV-3. Moreover, standard methods for producing attenuated viruses produce mutations at random sites and do not allow one to modify the HPIV genome at specific sites or to control the number of mutations that are introduced into genome.

Human parainfluenza viruses are enveloped, single-stranded, negative sense RNA viruses that are members of the paramyxovirus genus within the family Paramyxoviridae. Replication of the human parainfluenza viral genome (vRNA) is similar to that of other members of the Paramyxoviradae family. Upon infection of a cell, transcription is the major RNA synthetic event, resulting in the production of the viral mRNAs from the negative-sense genome, i.e., the vRNA. Later in infection a transition to RNA replication occurs, resulting in synthesis of a full-length, antigenomic, positive-sense RNA. which serves as the template for synthesis of additional negative-sense genomic RNA. Transcription and replication of the genomic RNA is dependent upon formation of a ribonucleoprotein complex (RNP) consisting of the 15462 nucleotide genomic RNA encapsidated by the nucleocapsid protein (NP), and the closely associated phosphoprotein (P), and the large (L) polymerase protein. Several host cell factors are also involved in the replicative cycle of HPIV.

The requirement for an intact RNP for HPIV has hindered analysis of HPIV transcription and replication in a cell-free system. Efforts to encapsidate HPIV-3 vRNA in vitro have failed, and unlike the positive sense RNA viruses, naked HPIV vRNA is not infectious. Moreover, there currently are no known systems for preparing recombinant HPIV, including recombinant infectious HPIV-3.

Accordingly, there is a need for new reagents, systems, and methods that enable one to produce a recombinant HPIV, particularly a recombinant, infectious HPIV-3. Recombinant systems that permit one to introduce one or more site-specific mutations into the genome of HPIV, particularly HPIV-3, are desirable. Recombinant systems which allow one to characterize the effect of site-specific mutations on the transcription or replication of human parainfluenza viral RNA and to identify the site specific mutations which lead to the production of attenuated HPIV are especially desirable.

### SUMMARY OF THE INVENTION

In accordance with the present invention a system for generating infectious, recombinant, human parainfluenza virus type 3 (HPIV-3) is provided. In one embodiment, the system comprises a clone comprising a nucleotide sequence that encodes a full-length, positive sense, anti-genome of HPIV-3, and at least one support clone comprising a nucleotide sequence that encodes the HPIV P protein and the HPIV L protein. In another embodiment, the system further comprises a support clone which comprises a nucleotide sequence that encodes the HPIV NP protein. Preferably, each of the clones in the system comprises an RNA polymerase promoter which is operatively linked to the respective HPIV nucleotide sequence contained within the clone.

The present invention also provides a clone which comprises a nucleotide sequence encoding the full-length, positive sense, anti-genome of HPIV-3. The clone also comprises an RNA polymerase promoter operatively linked to the HPIV-3 antigenome-encoding sequence. In a preferred embodiment, the clone further comprises a nucleotide sequence which encodes a ribozyme immediately downstream from the sequence encoding the HPIV-3 anti-genome.

The present invention also relates to a method of preparing recombinant HPIV-3 virus having site-specific mutations in the HPIV-3 genome. The method comprises preparing a clone comprising a modified HPIV-3 antigenome-encoding sequence; introducing the modified HPIV-3 clone and support clones which comprise nucleotide sequences encoding an HPIV-3 P protein, an HPIV-3 L protein, and, preferably, an HPIV-3 NP protein into host cells.; and culturing the host cells under conditions that allow for synthesis of the modified HPIV-3 antigenome and the L, P, and NP proteins of HPIV-3.

The ability to produce recombinant, HPIV-3 virus genetically engineered to contain site-specific mutations within the HPIV-3 genes and cis-acting elements expedites the study of all aspects of the virus replication cycle. Additionally, a system which permits production of recombinant HPIV that is genetically engineered to contain site-specific mutations within the HPIV-3 genome is useful for identifying attenuating parainfluenza genotypes and for developing a live vaccine for human parainfluenza virus.

### BRIEF DESCRIPTION OF THE FIGURES

Figure 1, depicts the DNA form of the nucleotide sequence of the HPIV-3 genome and shows the location of restriction sites, the leader sequence, the trailer sequence, and the protein encoding regions of the genome.
Figure 2 is a restriction map of the pOCUS-2^{™} vector.
Figure 3 is a restriction map of pMG(+) showing the location of the leader sequence, luciferase encoding region, and theT7 promoter and terminator.
Figure 4 is a restriction map of pHPIV-3 showing the location of the leader sequence and the protein encoding regions of HPIV-3.

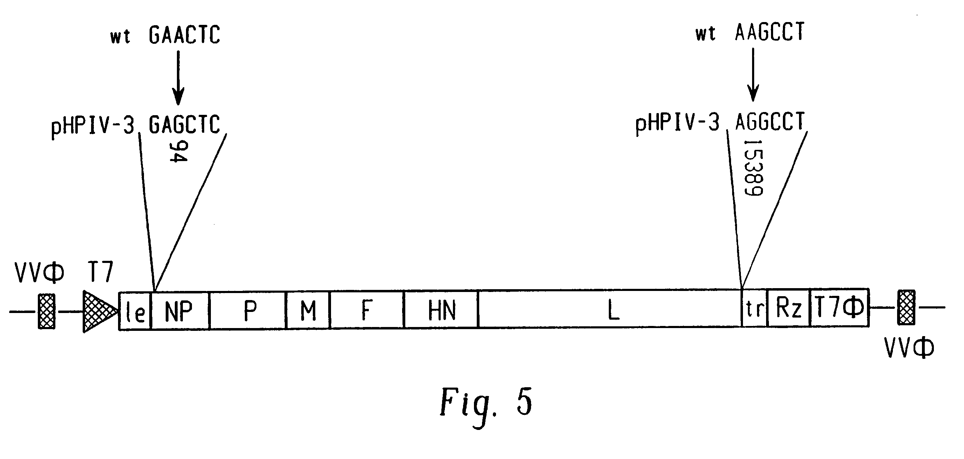

Figure 5 is a schematic depiction of the full-length infectious clone, pHPIV-3. VVφ, vaccinia virus polymerase stop signal (TTTTTNT); T7, T7 RNA polymerase promoter; Ie, HPIV-3 leader sequence; NP, P, M, F, HN and L are the HPIV-3 protein coding regions; tr, HPIV-3 trailer sequence; Rz, the hepatitis delta virus antigenomic ribozyme; T7φ, T7 RNA, polymerase terminator signal. Regions containing substitution mutations are expanded and shown above with the specific changes indicated. The A to G change at viral base 94 creates a *Sac*I site and the A to G change at viral base 15389 creates a *Stu*I site.

### DETAILED DESCRIPTION OF THE INVENTION

In accordance with the present invention a system for generating recombinant HPIV-3 is provided. The system comprises a clone comprising a nucleotide sequence, preferably a double-stranded DNA sequence, which encodes a full-length, positive sense anti-genome of HPIV hereinafter referred to as the "HPIV clone", and one or more support clones which comprise nucleotide sequences that encode an HPIV P protein and an HPIV L protein. The nucleotide sequences that encode the HPIV P protein and HPIV L protein may be within the same clone. However, for ease of manipulation, it is preferred that the nucleotide sequences that encode the HPIV P protein and the HPIV L protein be on separate clones. Preferably the HPIV clone comprises a sequence encoding an HPIV-3 antigenome. Preferably the support clone or clones encode a P protein and an L protein of HPIV-3. In another embodiment the system further comprises a support clone which comprises a nucleotide sequence that encodes the HPIV NP protein, preferably the HPIV-3 NP protein.

As used herein "clone" refers to double-stranded DNA that can be introduced into a cell and expressed. The clone may be in the form of a viral vector such as, for example, a vaccinia viral vector, or, preferably, in the form of a plasmid. Preferably, the HPIV clone and the support clones each comprise an RNA polymerase promoter, more preferably a T7 RNA polymerase promoter. Each of the RNA polymerase promoters is operatively linked to the corresponding HFIV encoding sequence in the clone. Thus, the RNA polymerase promoter on the HPIV clone is operatively linked to the HPIV sequence and the RNA polymerase on the support clones are operatively linked to the sequence or sequences encoding the respective HPIV protein. Preferably, the plasmids comprising the clone also comprise an origin of replication, particularly a bacterial origin of replication.

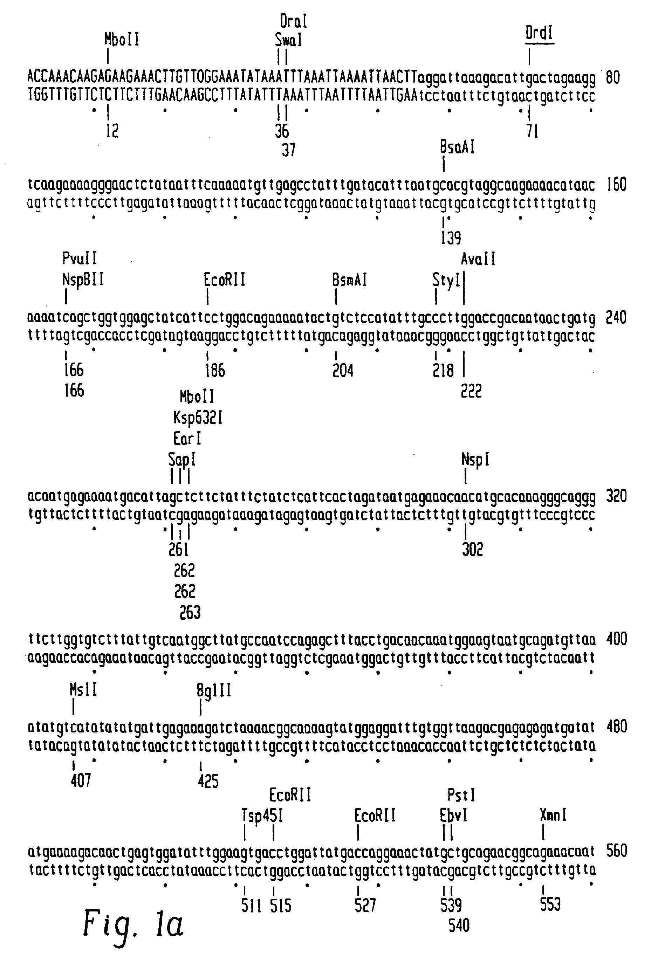

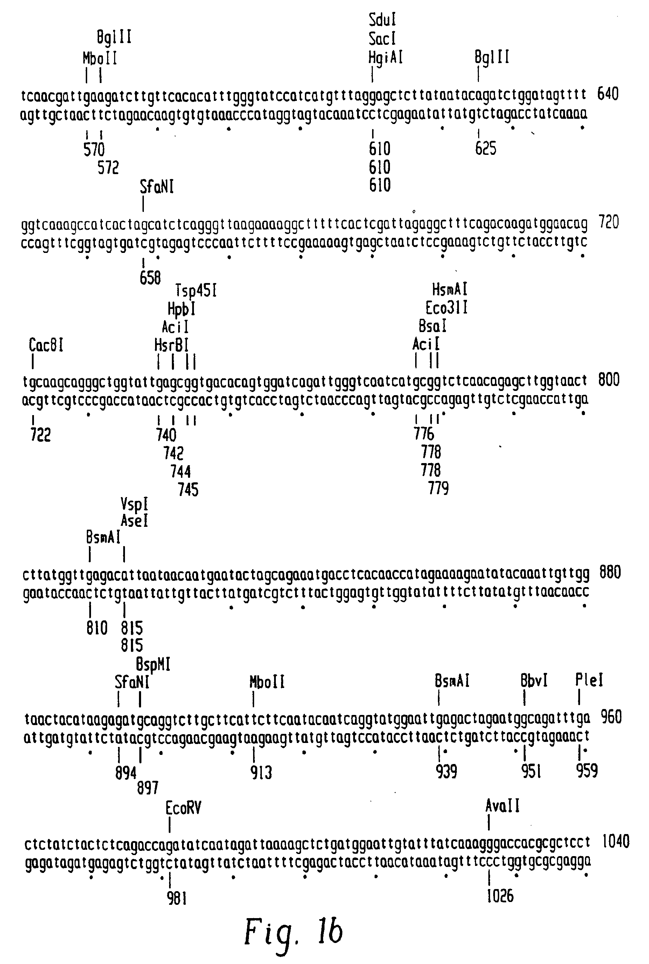

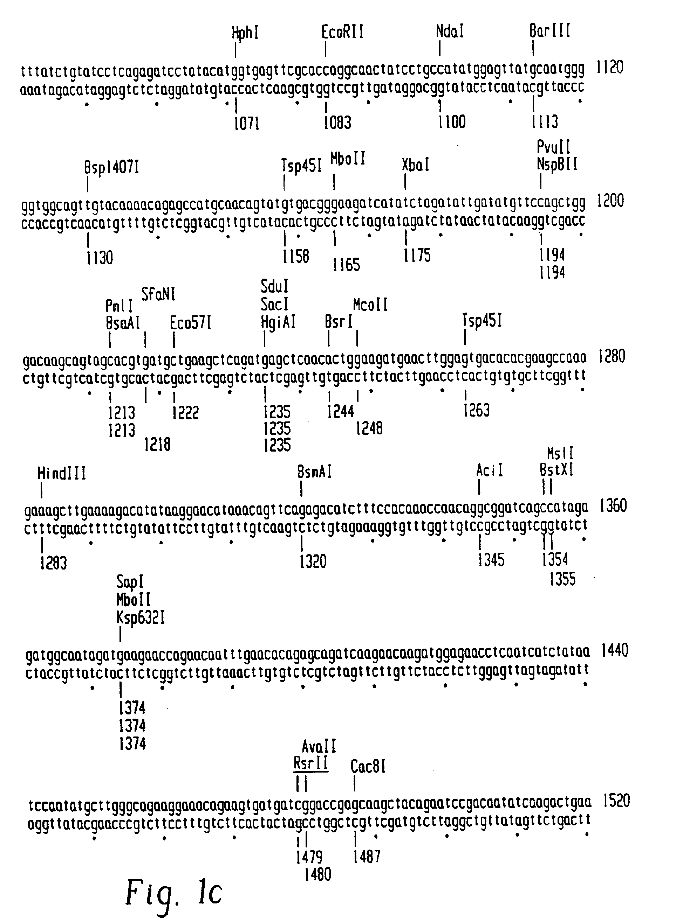

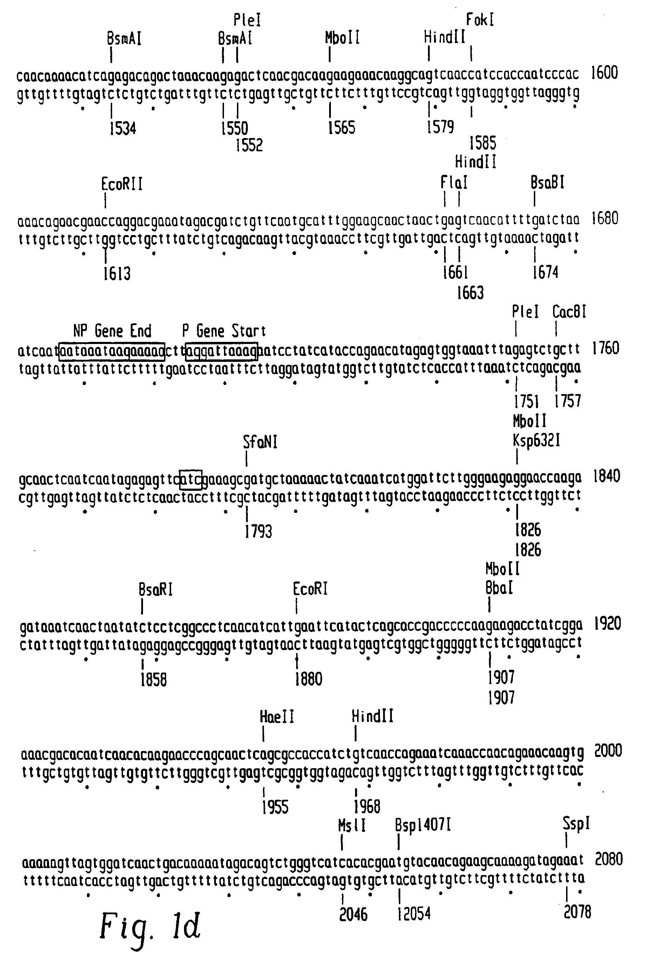

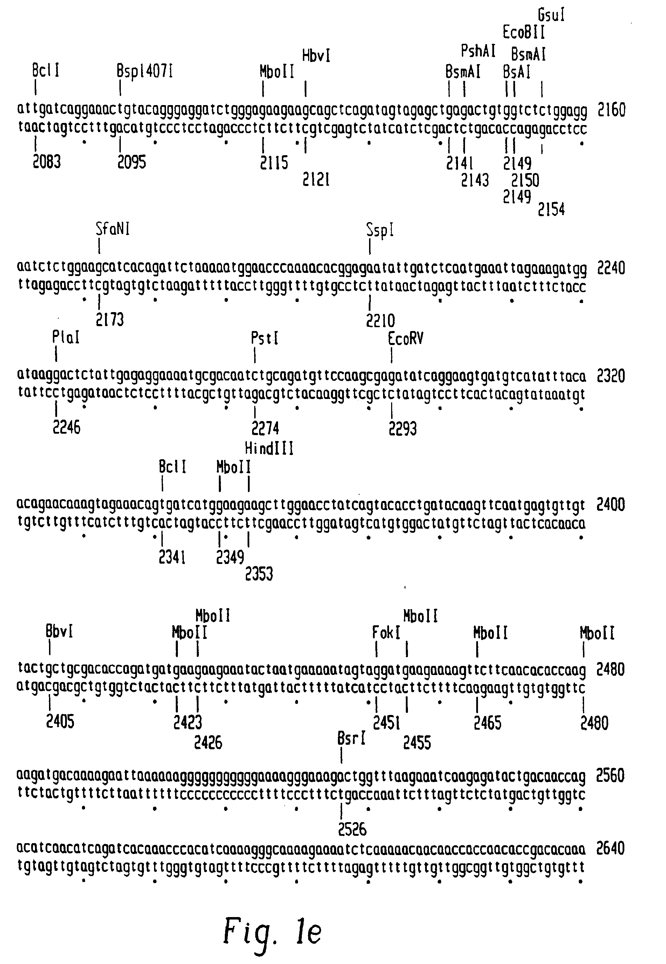

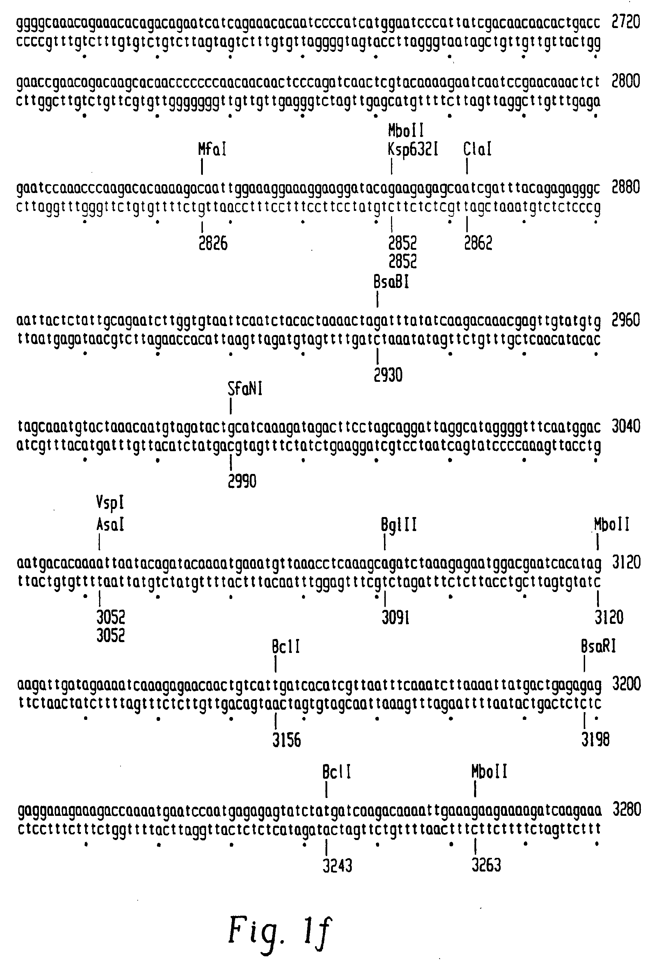

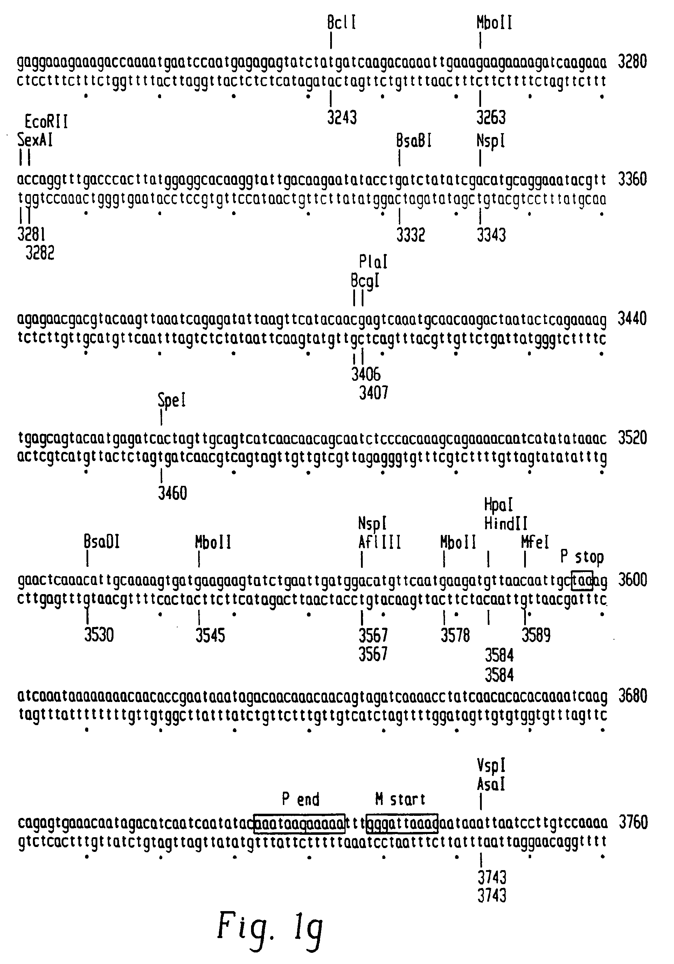

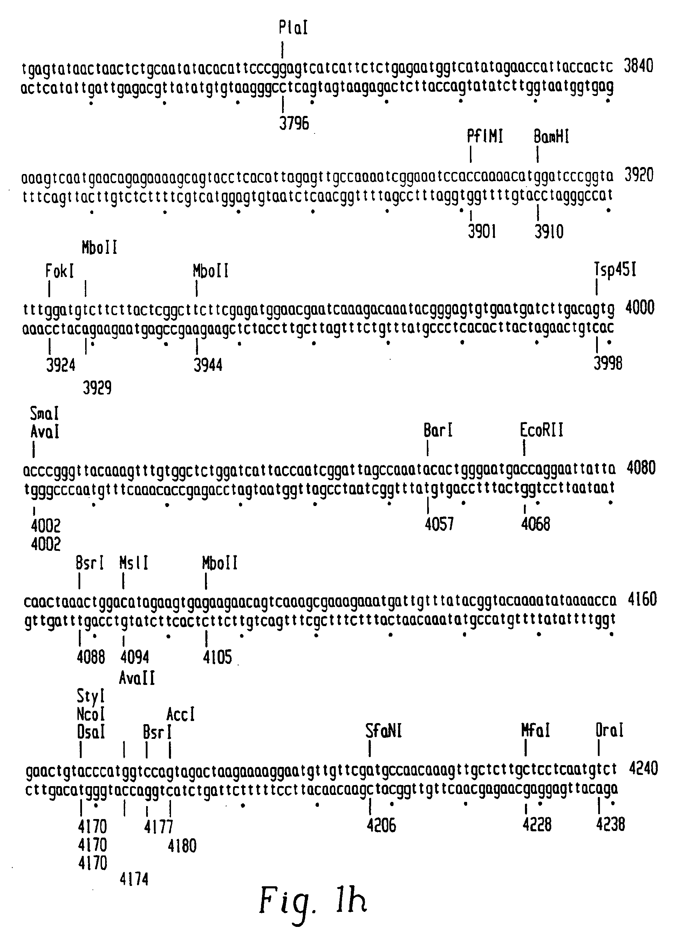

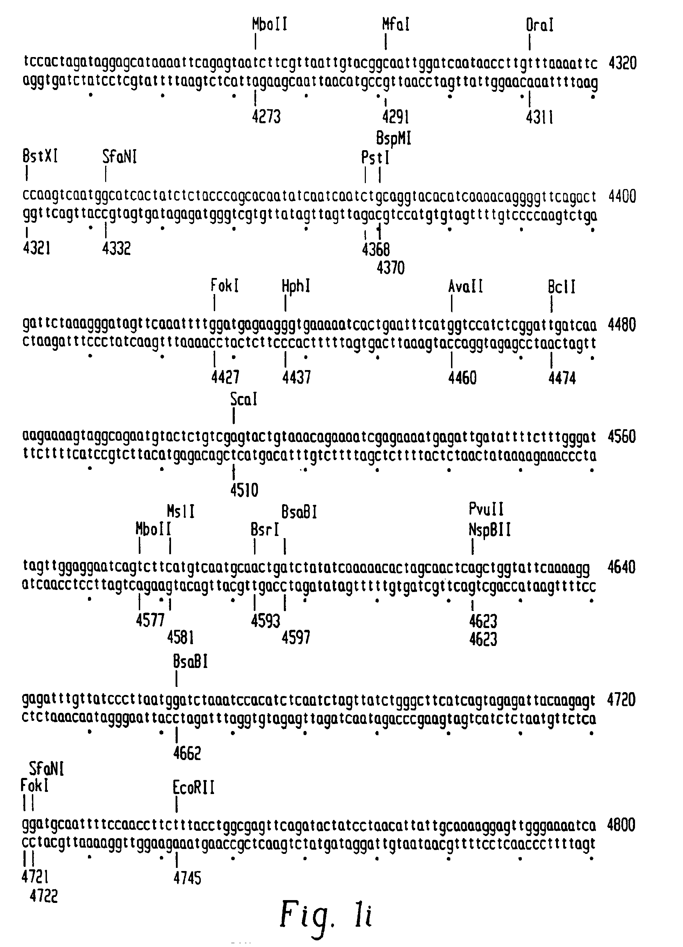

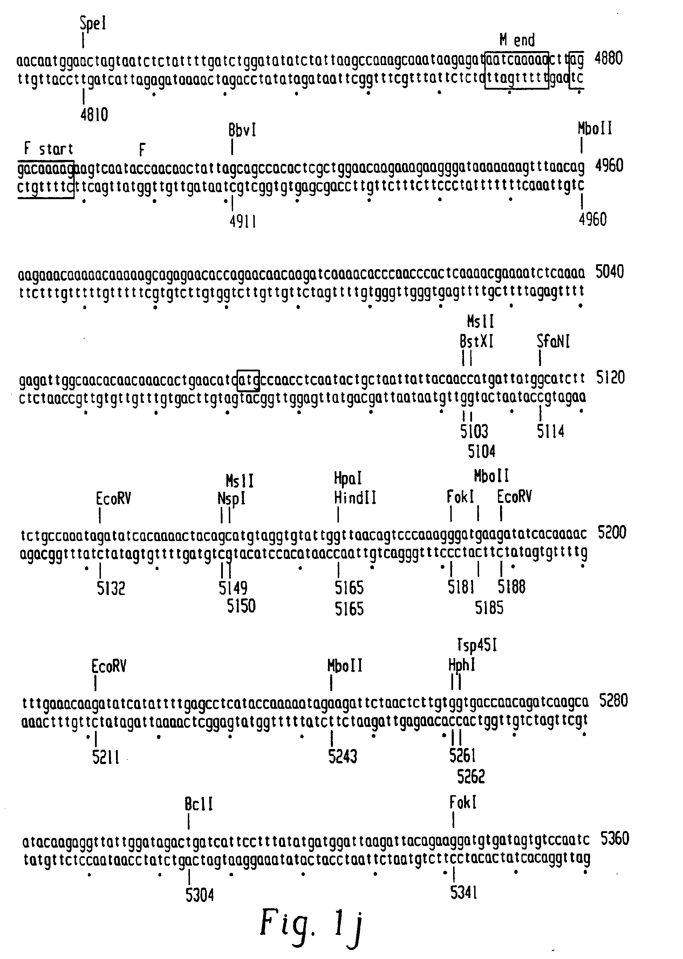

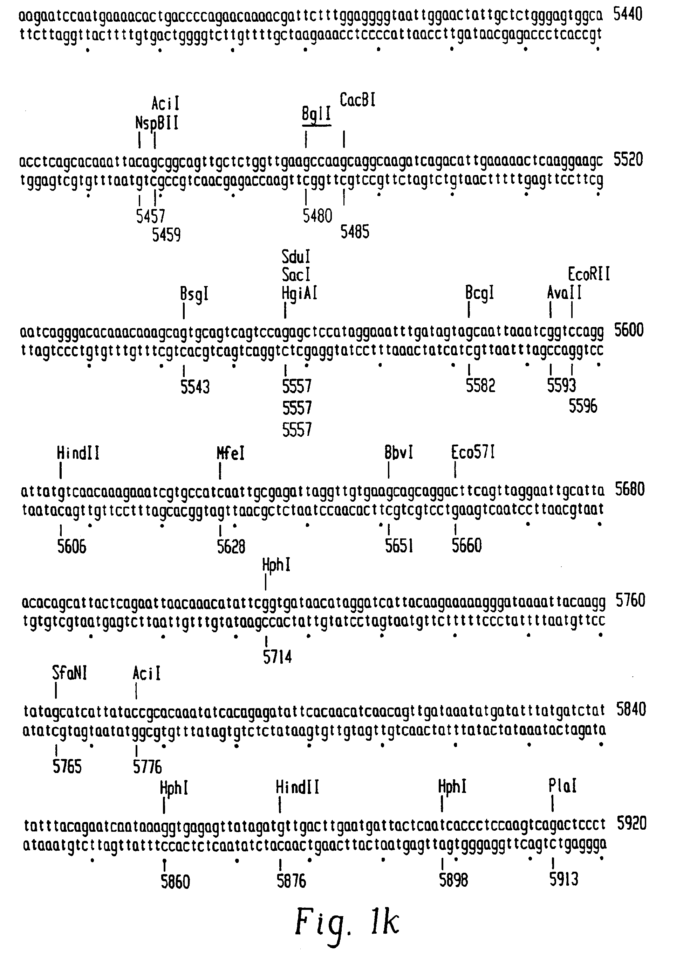

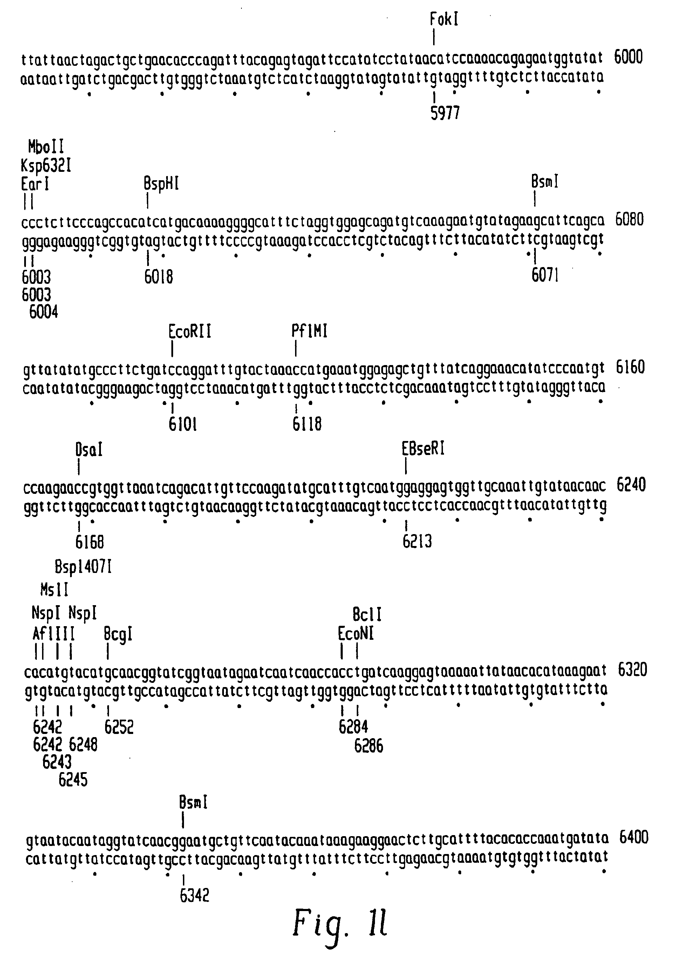

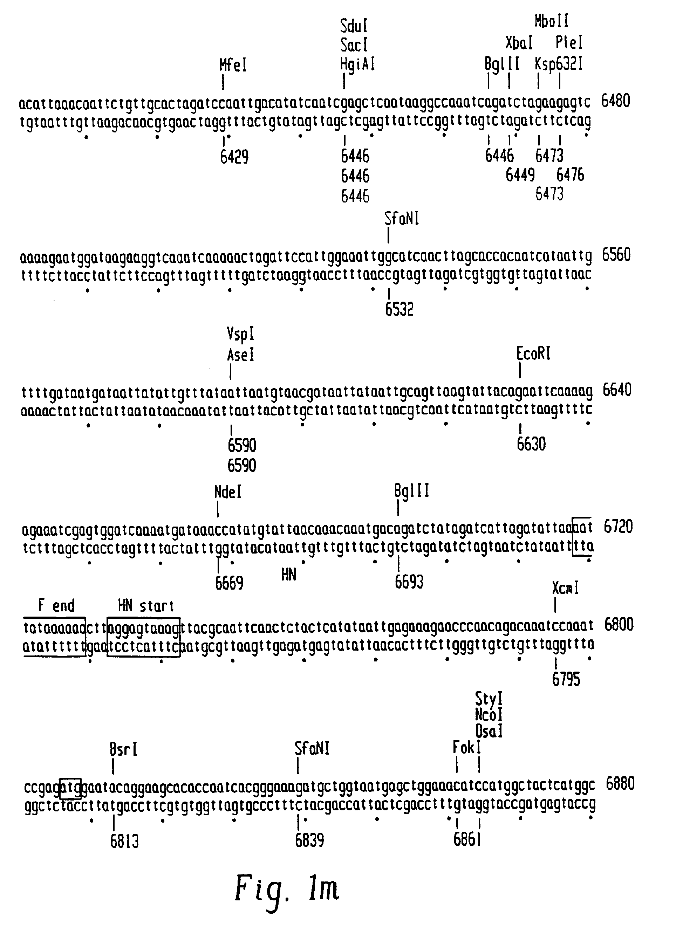

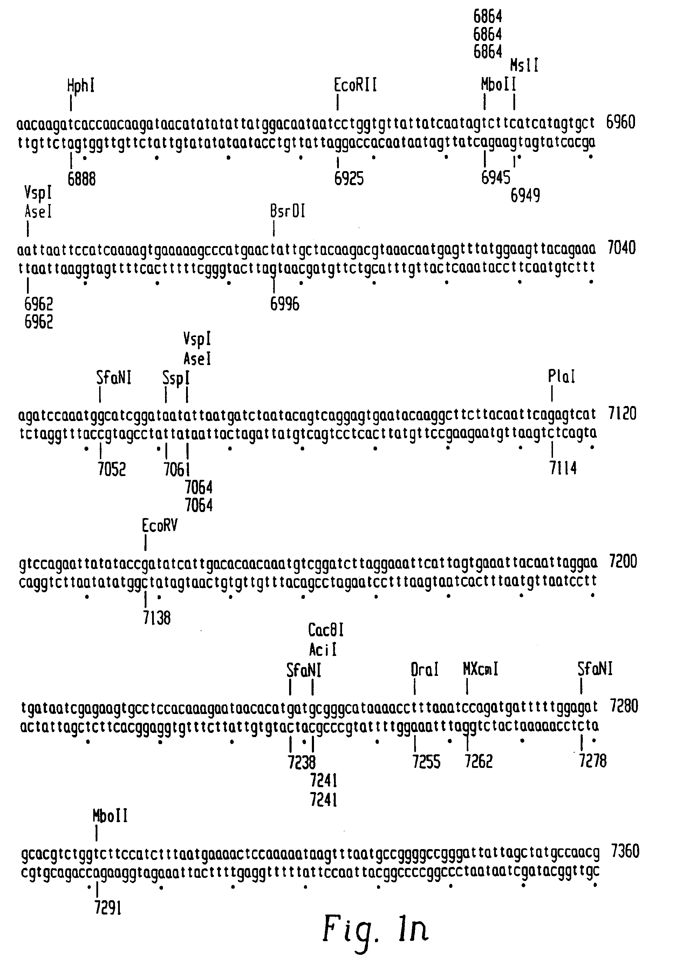

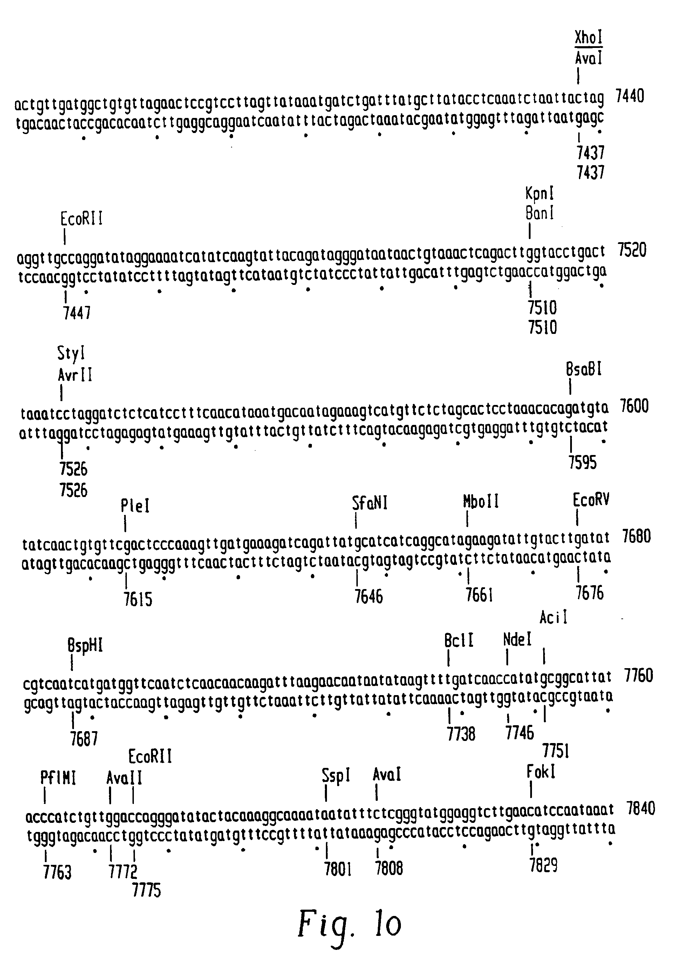

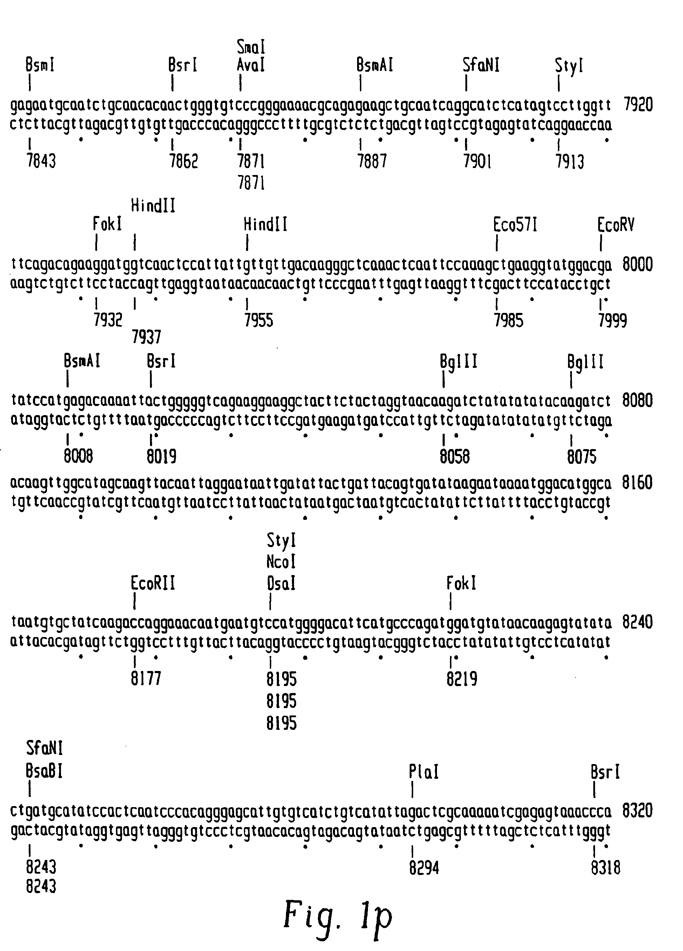

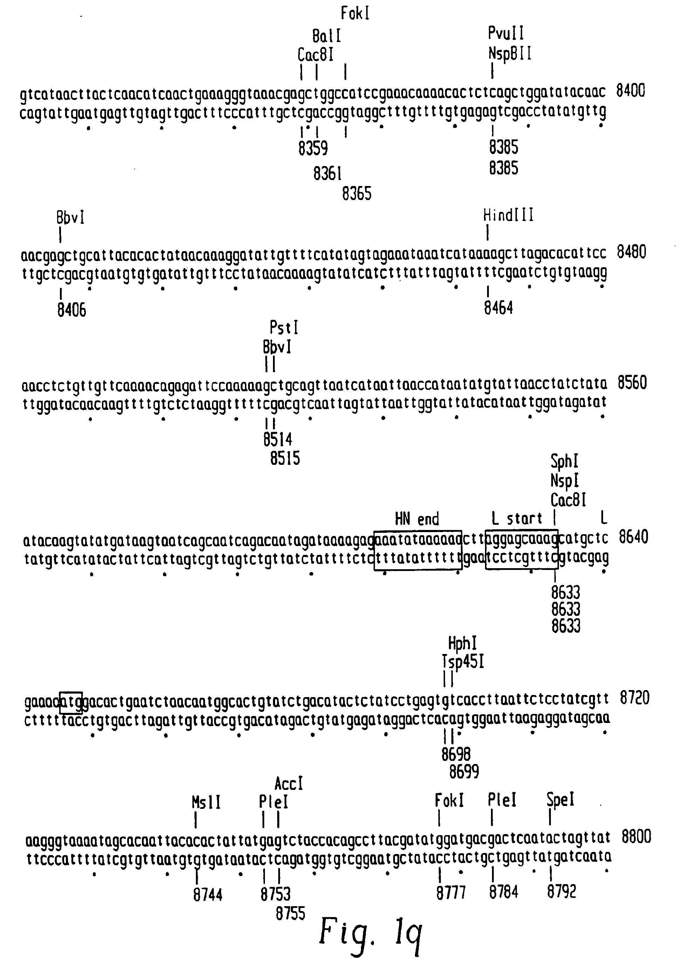

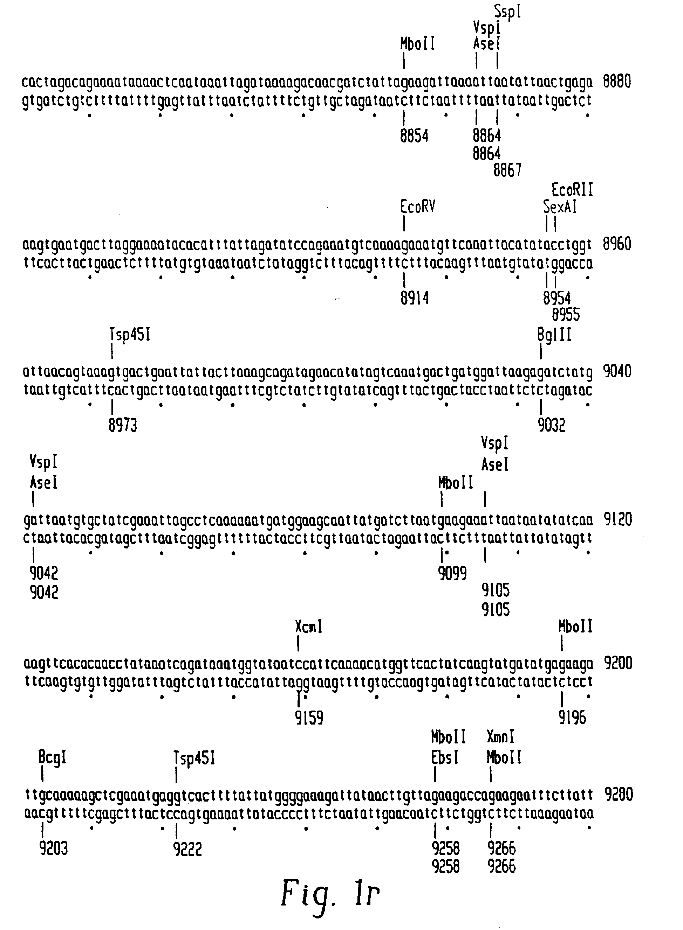

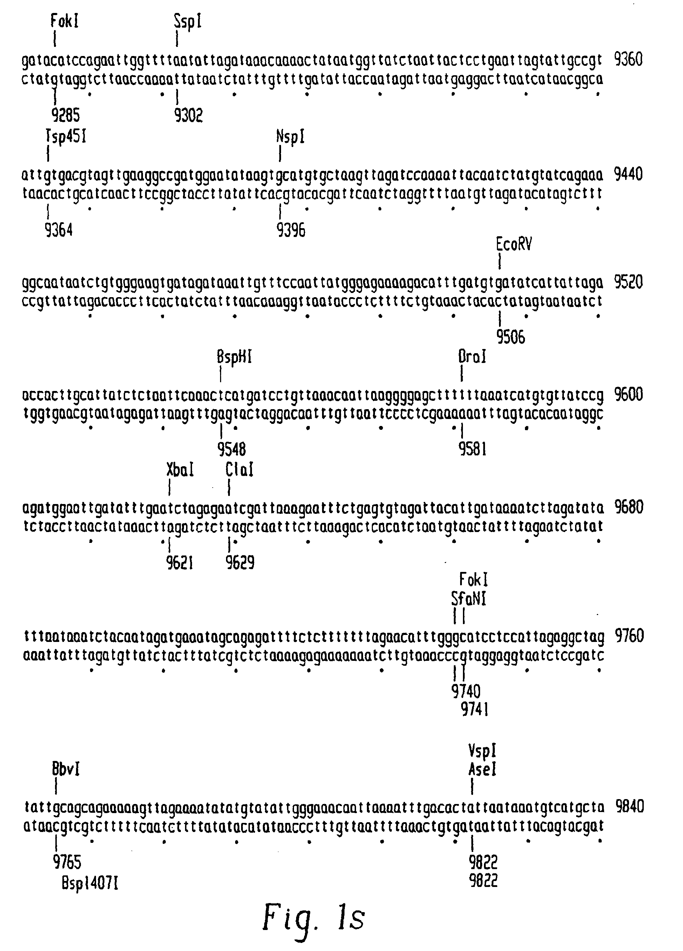

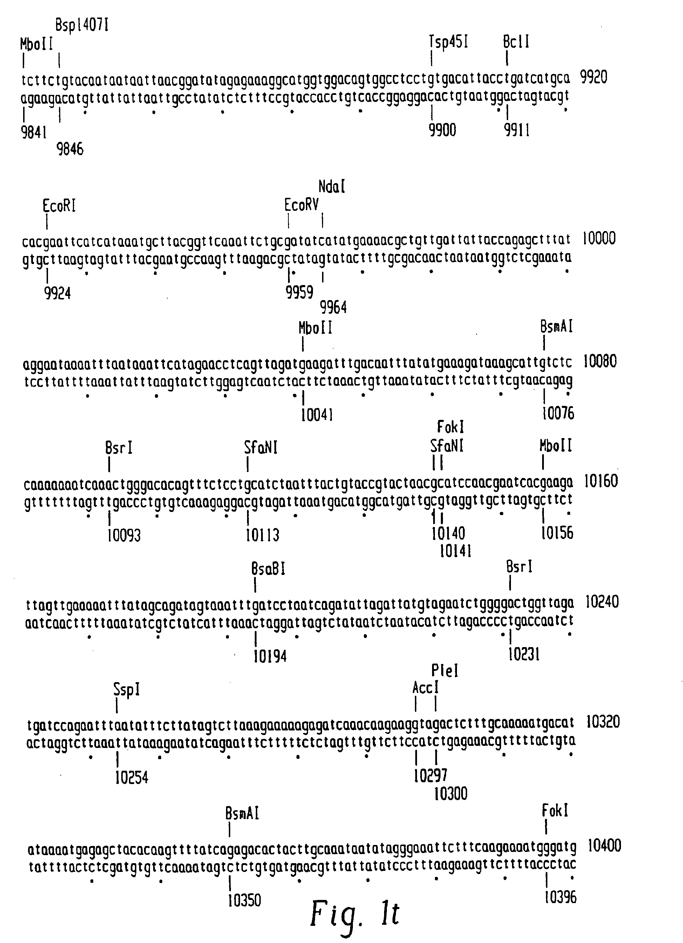

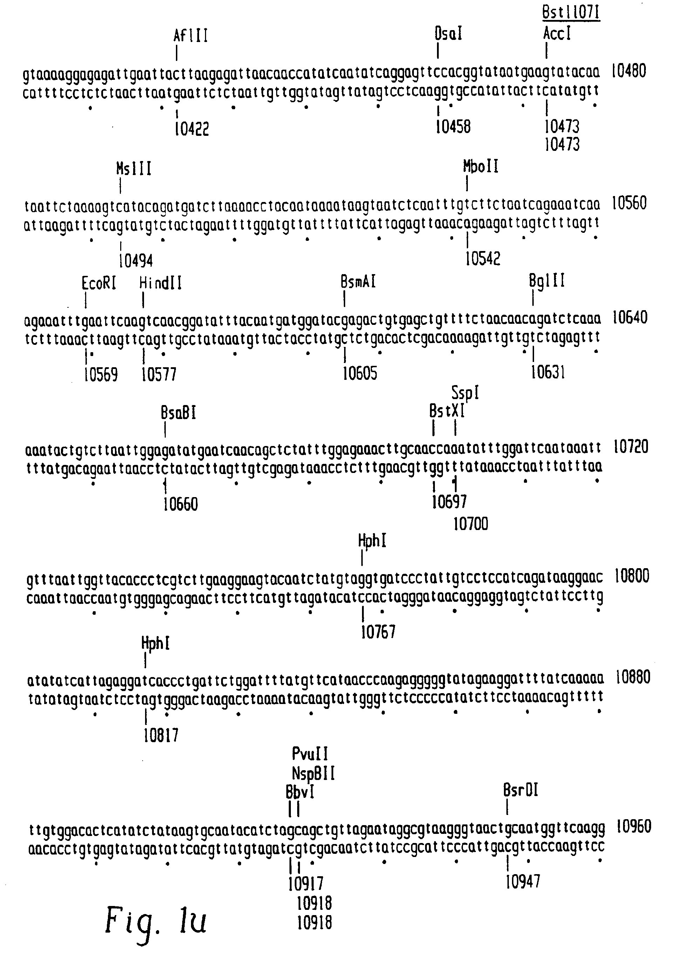

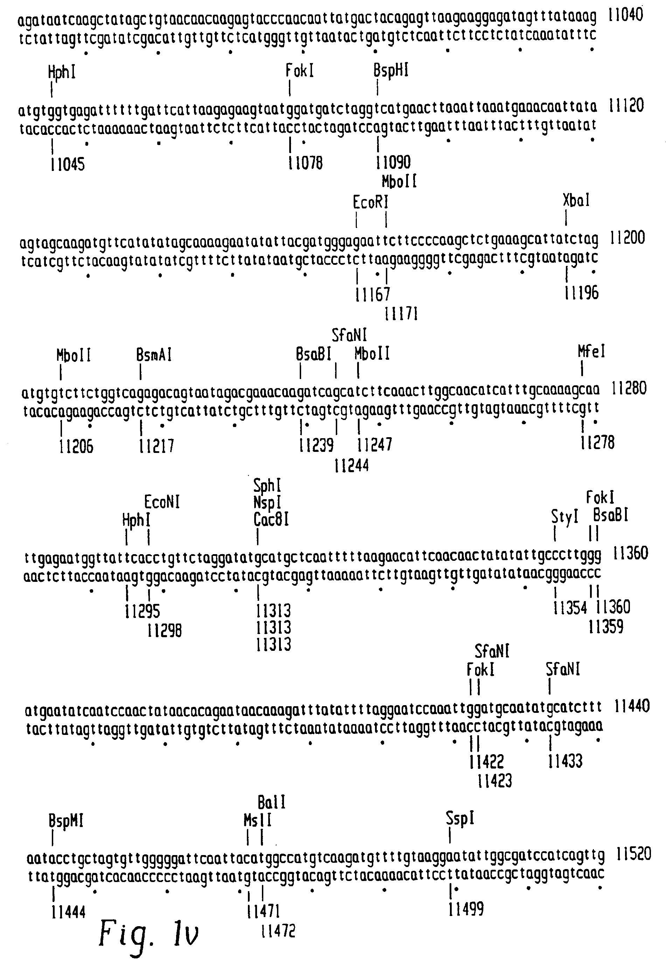

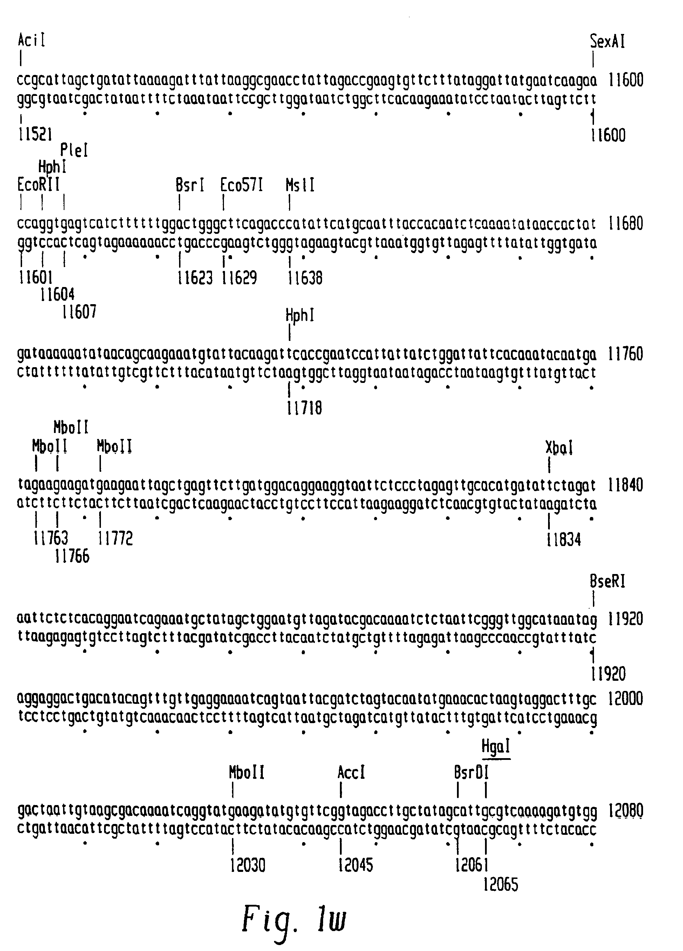

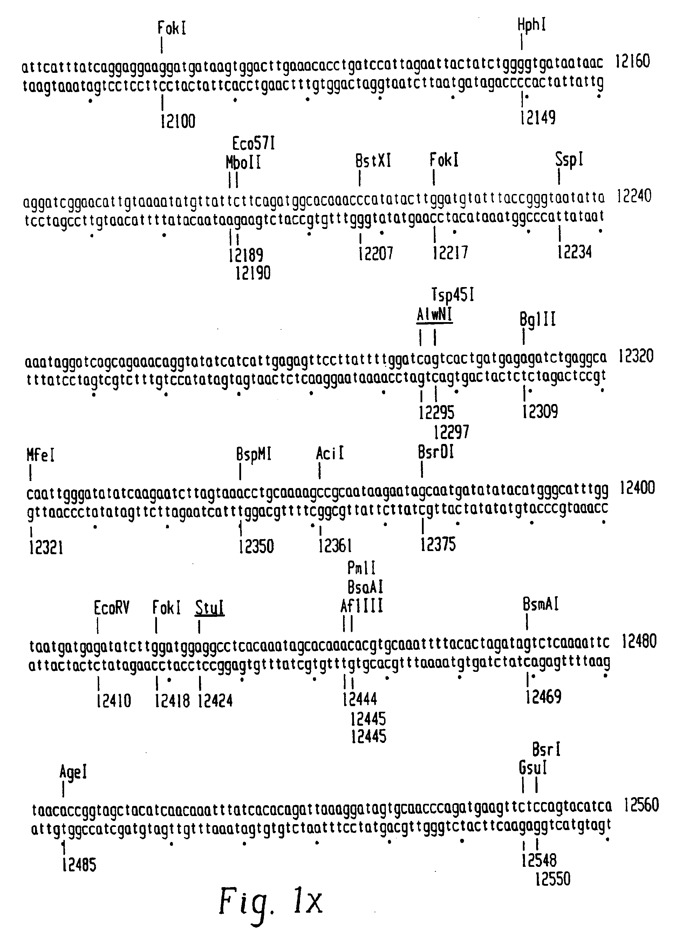

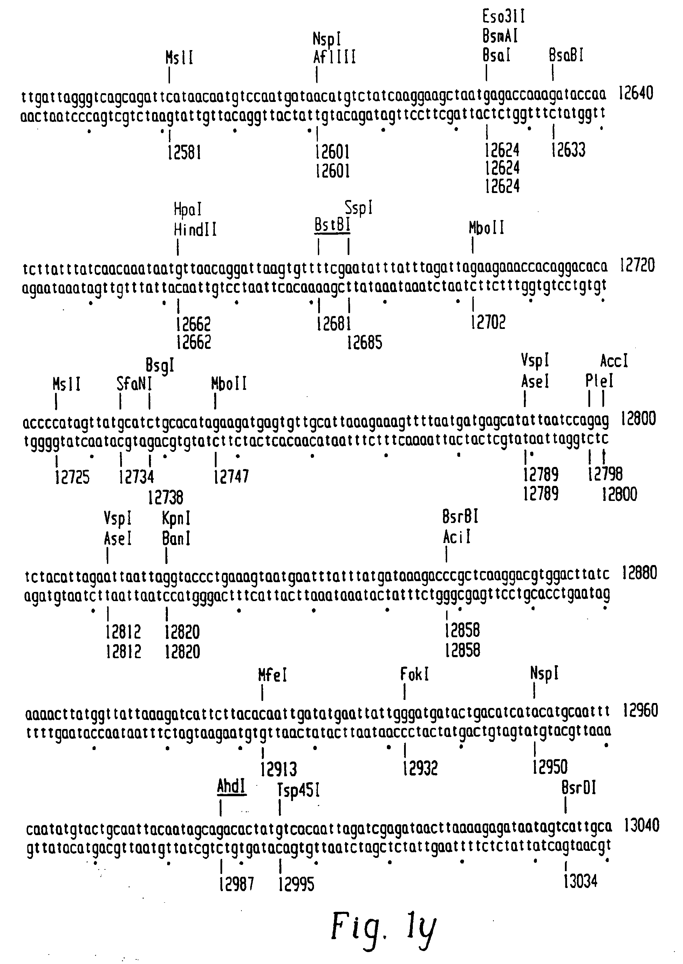

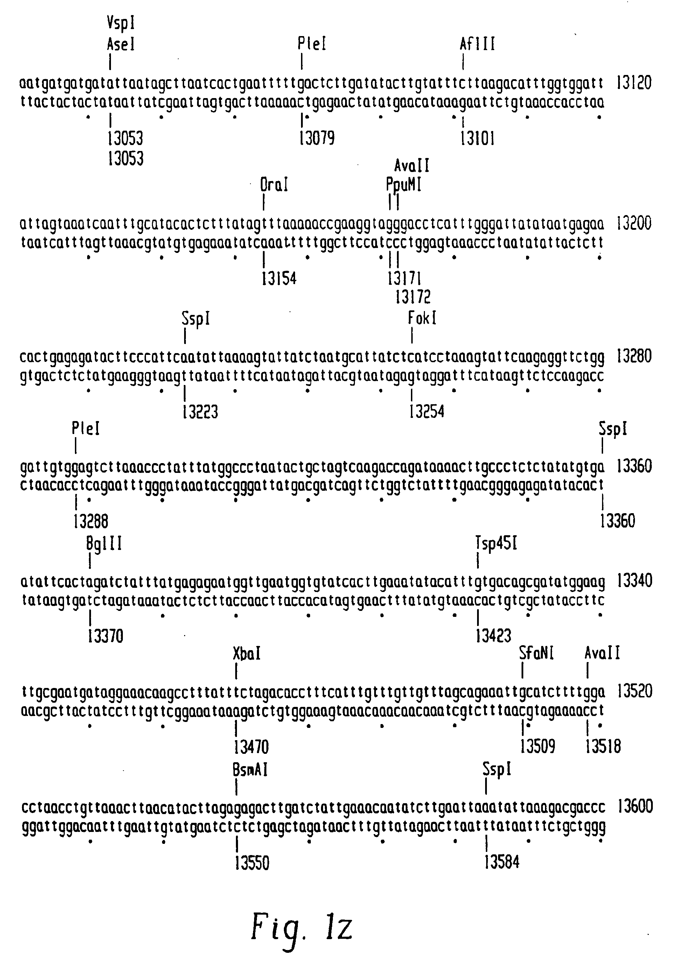

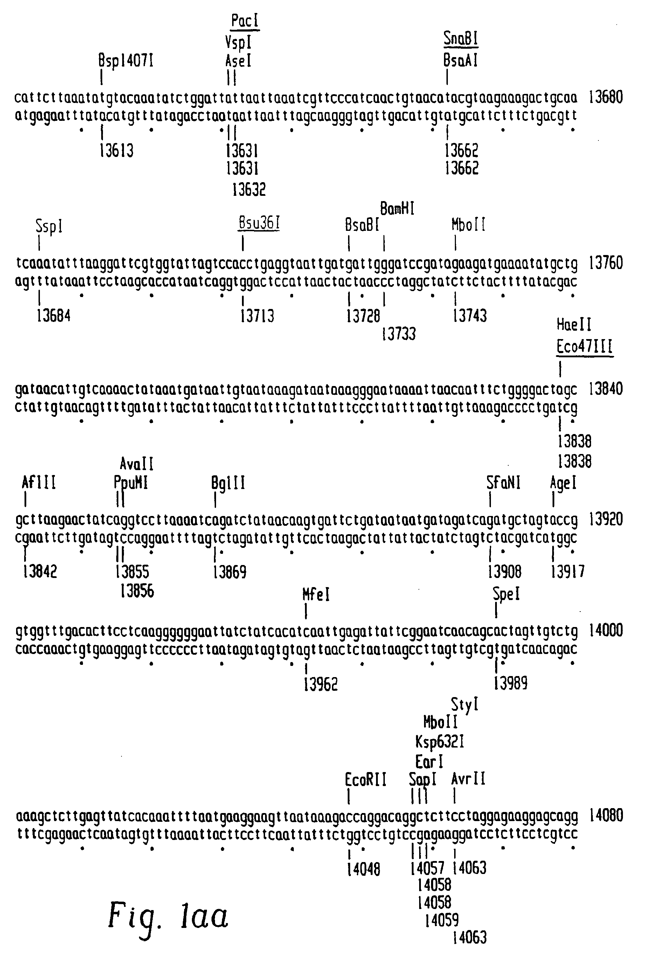

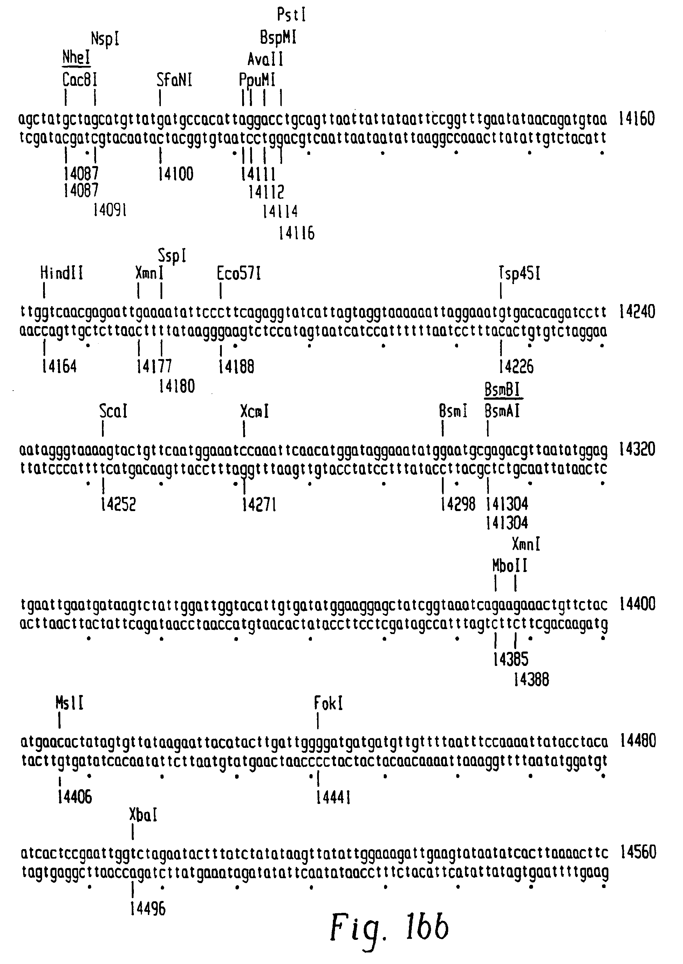

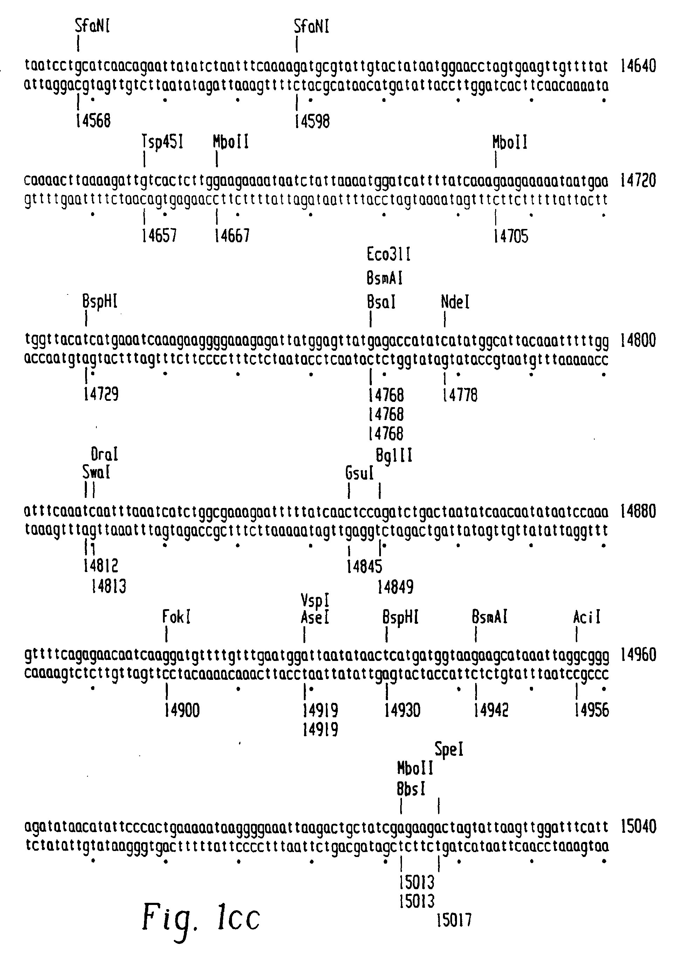

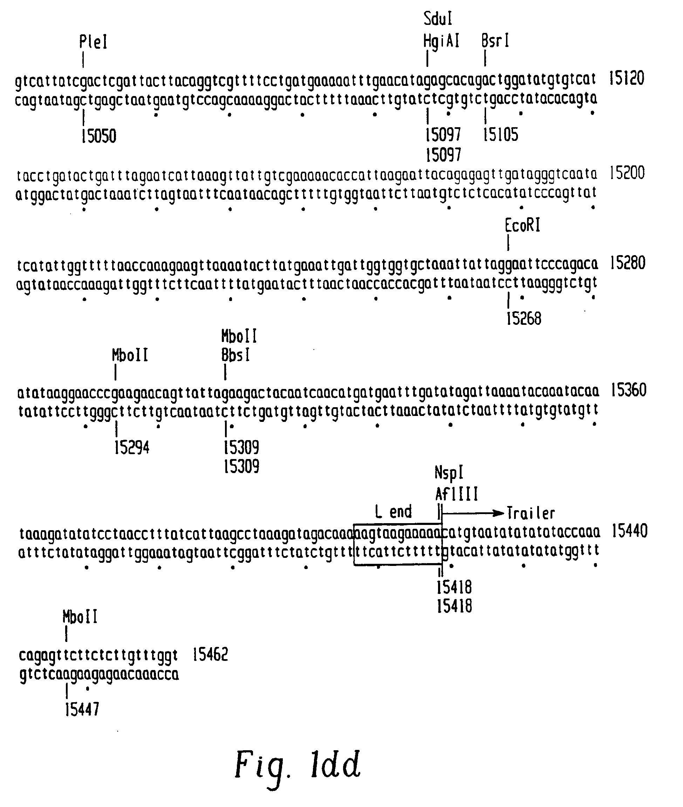

The present invention also provides a clone which comprises a nucleotide sequence encoding the anti-genomic sequence of HPIV-3, hereinafter referred to as the "HPIV-3 clone". Preferably, the HPIV-3 clone encodes a full-length antigenomic sequence of HPIV-3. As used herein "full-length" means that the anti-genomic sequence is complementary to the entire negative sense, genomic sequence of HPIV-3 extending from the 3' nucleotide of the leader sequence through the 5'nucleotide of the trailer sequence of the HPIV-3 genome. The DNA form of the full-length, genomic sequence of HPIV-3, SEQ ID NO:1, is shown in Fig. 1. In addition to the leader and trailer sequences, the HPIV-3 clone contains sequences encoding the HPIV-3 proteins N, P, M, F, HN, and L, as well as the cis-acting elements. The HPIV-3 clone may encode a wild-type HPIV-3 antigenome sequence or a modified HPIV-3 antigenome having one or more mutations contained therein. The mutation may be in the form of a foreign gene which is inserted into the HPIV-3 antigenome-encoding sequence. Preferably, the mutations are substitutions of one or more nucleotides, deletions of 6 to 12 nucleotides, or additions of 6 to 12 nucleotides in the HPIV-3 antigenome-encoding sequence. More preferably, the modified HPIV-3 clone contains substitutions either in the genes or the cis-acting elements, or both of the HPIV-3 antigenome-encoding sequence.

Preferably, the HPIV-3 clone is a plasmid that comprises a nucleotide sequence which encodes a ribozyme, more preferably the hepatitis delta virus antigenomic ribozyme, immediately downstream from the HPIV antigenome-encoding sequence. Following transcription of the clone, the ribozyme cleaves the ribozyme from the HPIV antigenome to provide a replication competent 3' end on the antigenome. More preferably, the HPIV-3 clone also comprises an RNA polymerase terminator following the ribozyme sequence. In one embodiment, the HPIV-3 clone is the plasmid pHPIV-3 depicted in Fig. 5, which plasmid was deposited with the American Type Culture Collection, 12301 Parklawn Drive, Rockville, MD 20852, USA, on May __, 1998 and has Accession Number PTA-722.

The present invention also relates to a method of preparing recombinant HPIV, particularly HPIV-3, using the above described system. The method comprises introducing an HPIV clone and the support clones which encode HPIV P protein and HPIV L protein, into host cells, preferably human cells; culturing the host cells under conditions that allow for formation of an HPIV anti-genomic transcript, synthesis of the HPIV genome (vRNA) and the HPIV proteins L, P, and NP, and formation of a recombinant HPIV; and recovering the recombinant HPIV from the culture. Preferably the host cells which are transfected with the HPIV clone and support clones, contain an RNA polymerase that corresponds to the RNA polymerase promoter that is operatively linked to the HPIV sequences in the HPIV clone and the support clones. In a preferred embodiment, a support clone comprising the nucleotide sequence which encodes the HPIV-NP protein operatively linked to an RNA polymerase promoter is also introduced into the cells. Preferably, the host cells are infected with a viral recombinant, preferably a vaccinia virus recombinant, which expresses the RNA polymerase, more preferably the T7 RNA polymerase, prior to or in combination with transfection with the HPIV clone and support clone or clones. When such cells are infected with the vaccinia virus recombinant, it is preferred that the HPIV-3 clone also comprise a vaccinia virus RNA polymerase terminator upstream of the T7 RNA polymerase and a vaccinia virus RNA polymerase terminator downstream of the T7 RNA polymerase terminator.

The present invention also relates to a method of introducing site-specific mutations into the genome of a recombinant HPIV-3. The method comprises preparing a a modified HPIV-3 clone comprising one or more mutations in the sequence which encodes the HPIV-3 anti-genome; introducing the modified HPIV-3 clone and support clones comprising sequences which encode HPIV-3 P protein and HPIV-3 L protein, and preferably, HPIV-3 NP protein into host cells; and culturing the host cells under conditions that allow for formation of a modified HPIV-3 antigenomic transcript and synthesis of the HPIV-3 L, P, and NP proteins. The modified HPIV-3 clone and the support clones contain an RNA polymerase promoter that is operatively linked to the HPIV-3 protein-encoding sequences. The host cells contain within the cytoplasm thereof an RNA polymerase that corresponds to the RNA polymerase promoter on the modified HPIV-3 clone and the support clones.

Preferably, the modified HPIV-3 clone, containing one or more mutations therein, is made by conventional PCR techniques using an HPIV-3 clone as a template. The mutations are made in the cis-acting elements of the HPIV-3 sequence or in an HPIV-3 protein encoding sequence. Preferably the mutation is made in the L protein-encoding sequence. If mutations are made in the HPIV-3 protein-encoding sequences of the HPIV-3 clone, it is preferred that a similar type of mutation be made in the same site in the protein encoding sequence of the corresponding support clone. For example, if a mutation is made at a specific site in the L protein-encoding sequence of the HPIV-3 clone, it is preferred that the same mutation be made at the same site in the L protein-encoding sequence of the L protein-encoding support clone. Such method is useful for identifying mutations that block the synthesis of viral particles or result in the production of non-infectious or non-virulent HPIV-3.

To determine whether the mutated viruses produced by the above-described method are non-virulent, i.e., attenuated, the mutated viruses are first tested in vitro to determine whether the mutation has resulted in a slower growing phenotype, i.e., the mutated virus grows more slowly in tissue culture than the wild-type virus. The mutated viruses which exhibit this phenotype are then examined in vivo, by injection into an animal, such as the cotton rat, which is good experimental model for parainfluenza virus. The infected animals are then examined to determine if they are producing antibodies to HPIV-3 and to determine if there is a reduction in the severity of symptoms as compared to animals infected with wild-type virus.

The ability to produce recombinant HPIV-3 virus genetically engineered to contain specific alterations within the HPIV-3 genes and cis-acting elements expedites the study of all aspects of the virus replication cycle. Additionally, a system which permits production of recombinant HPIV that is genetically engineered to contain specific alterations within the HPIV-3 genes is useful for identifying attenuating parainfluenza genotypes and for developing a live vaccine for human parainfluenza virus.

The following examples of methods of preparing a full-length cDNA clone of HPIV-3 and methods of preparing a modified or mutated, infectious, recombinant HPIV -3 are for purposes of illustration and are not intended to limit the scope of the invention.

### Example 1. Construction of a Full-Length cDNA Clone of HPIV-3.

The construction of a full-length infectious clone of HPIV-3 containing mutations at specific sites was achieved by a two-step process. The initial step was the generation of a minireplicon which contained the positive sense leader portion region and trailer regions of HPIV-3. The second step involved the insertion of RT-PCR fragments derived from HPIV-3 genomic RNA into the minireplicon. The positive-sense minireplicon contained the following: A T7 promoter which directed the synthesis of two non-viral G residues, followed by the positive-sense leader region of HPIV-3, a portion of the NP 5' UTR (to viral base 97), the luciferase gene, a portion of the L 3'UTR (starting at viral base 15387) and trailer sequences of HPIV-3. The full-length nucleotide sequence of HPIV-3 genome and the location of the leader sequence, trailer sequence and protein-encoding regions is shown in Figure 1. The hepatitis delta virus antigenomic ribozyme followed to effect precise cleavage after the 3 terminal HPIV-3 specific base. A T7 RNA polymerase terminator was also incorporated into the replicon followed the ribozyme sequence. Additionally, vaccinia virus polymerase termination signals were inserted immediately upstream and downstream of the aforementioned sequences. During the construction, single base changes were created in the regions encoding the NP 5' UTR and the L 3 UTR. An A to G change at viral base 94 and the A to G change at base 15389 created SacI and StuI sites, respectively, which served as genetic tags to identify virus as being of recombinant origin.

The vector pOCUS-2 (Novagen) was chosen as the starting plasmid for preparing the mini-replicon [pPIV3-MG(+)], because of its small size (1930 bp). It is believed that the use of a small starting plasmid may increase the stability of the full-length clone.

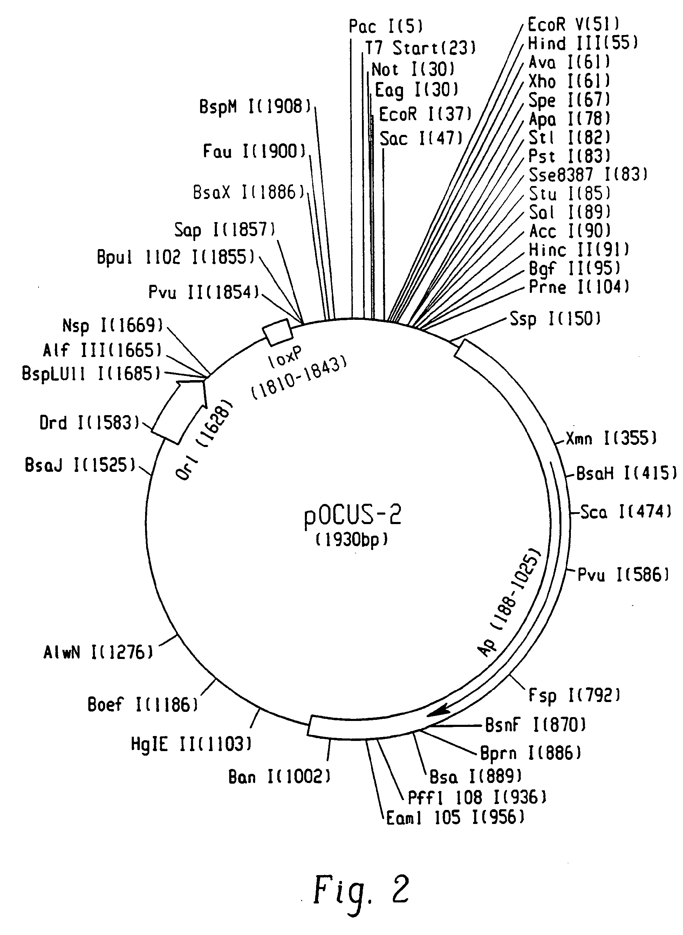

The mini-replicon was constructed by generating PCR products encoding the leader and trailer regions flanked by a T7 promoter and hepatitis delta virus antigenomic ribozyme, respectively. The primers used for synthesis of the T7 promoter/leader region were: 5'-TAGTCGGCCCTAATACGACTCACTATAGGACCAAACAAGAGAAGAA ACT-3', SEQ ID NO:2, and 5'-GAAATTATAGAGCTCCCTTTTCT-3', SEQ ID NO:3. The first primer encodes an EagI site and the T7 promoter (underlined) and the second primer introduced an A to G base change at viral base 94, (bold) within the 5' untranslated region (UTR) of the NP mRNA, which creates a SacI site. The template for this reaction pHPIV3-CAT, described in De, B.P. and A.K. Banerjee- (1993.) Rescue of synthetic analogs of genome RNA of human parainfluenza virus type 3. Vir. 196:344-348. The resulting PCR product was cloned into the EagI and SacI sites of pOCUS-2, which is depicted in Figure 2. The primers used for synthesis of the trailer/ribozyme region were: 5'-TAAGGCCTAAAGATAGACAAAAAGTAAGAAAAACATGTAATATATATA TACCAAACAGAGTTCTTCTCTTGTTTGGTGGGTCGGCATGGCATCTC-3', SEQ ID NO:4, and 5'-CTGGGTACCTCCCTTAGCCATCCGAGT-3', SEQ ID NO:5. The first primer contains sequence from the 3' UTR of the L mRNA, through the trailer, and primes synthesis of the ribozyme (underlined). Also, an A to G change at viral base 15389 (bold), which creates a StuI site within the 3'UTR of the L mRNA is encoded by this primer. The second primer encodes the 3' end of the ribozyme (underlined) and a BgIII site. The template for this PCR reaction was pSA1, a plasmid containing the ribozyme sequence, as previously described in Perrotta, A. T. and M. D. Been. 1991. The pseudoknot-like structure required for efficient self-cleavage of hepatitis delta virus RNA. Nature 350:434-436. The PCR product derived from this reaction was cloned into the StuI and BglII sites of pOCUS-2. The leader and trailer regions were combined into a single clone by transferring the EagI/PstI fragment of the T7/leader clone into the PacI/PstI sites of the trailer/ribozyme clone.

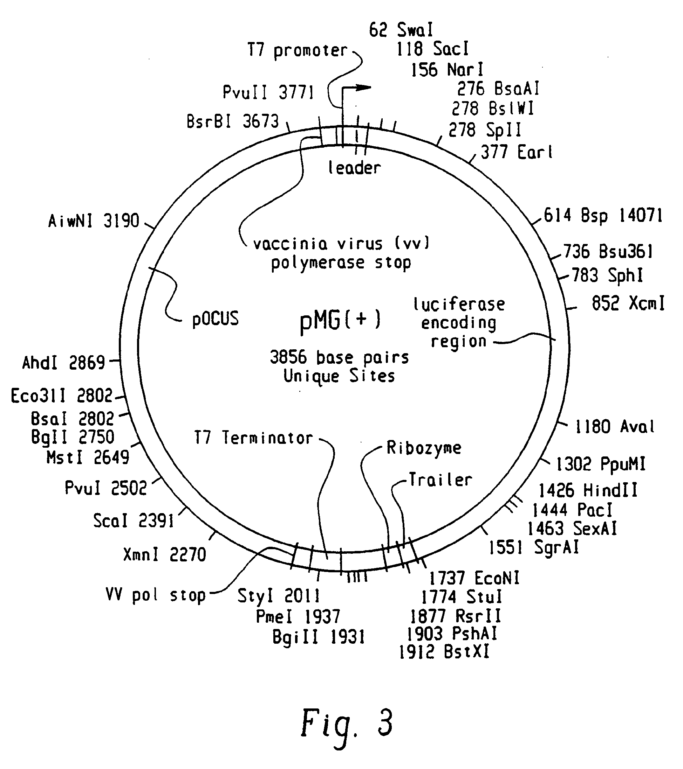

To prevent possible interference by transcription from cryptic vaccinia virus promoters, vaccinia virus polymerase transcription stop signals (TTTTTNT) were inserted upstream and downstream of the replicon near PvuII and SspI sites within pOCUS-2. A T7 transcription termination signal was removed from pET-17b by digesting with BlpI and BspEI, and inserted into the SspI site (blunted with T4 DNA polymerase) of pOCUS-2. A luciferase reporter gene was then inserted into the SacI and StuI sites to create pPIV3-MG(+), which is schematically depicted in Figure 3.

To generate the full-length HPIV-3 clone, five RT-PCR products were generated from HPIV-3 virion RNA and cloned. These fragments were subsequently inserted into pPTV3-MG(+), replacing the luciferase coding sequences, to create pHPIV-3, the full-length clone. The five RT-PCR products were generated from HPIV-3 strain 47885 virion RNA which was obtained from Robert Chanock, at the National Institues of Health. These PCR products, encompassing the remainder of the HPIV-3 genome, were identified by restriction enzyme analysis and cloned, either in pUC19 or pOCUS-2, and then inserted into pPIV3-MG(+).

The first PCR product containing viral bases 83 to 2721 was inserted into the SmaI site of pUC19. The 83/2721 clone was then digested with SacI and XmnI, removing viral bases 94 to 553 which were inserted into the SacI and SphI (blunt with T4 DNA polymerase) of pPIV3-MG(+), The 83/2721 clone was then digested with PstI to remove a fragment containing viral bases 540 to 2274, which was then inserted into the PstI site of the pPIV3-MG(+) clone containing the 94/554 fragment. The second PCR product encompassing viral bases 13395 to 15397 was cloned into the SmaI site of pUC19. This 13395/15397 clone was then digested with StuI and PacI and the resulting fragment containing viral bases 13632 to 15381 was inserted into the Stul and PacI sites of the pPIV3-MG(+) clone containing viral sequence to base 2274. The resulting clone contained viral bases 1 to 2274 and 13632 to 15462 in the pPIV3-MG(+) context.

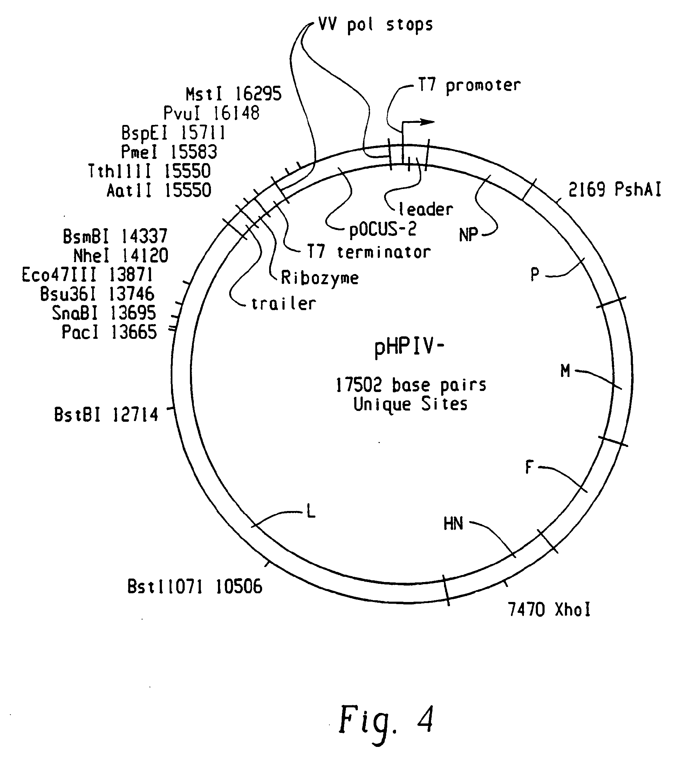

The third PCR product containing viral bases 7403 to 11513 was digested with BspMI (blunted with T4 DNA polymerase) and XhoI to produce a fragment containing bases 7437 to 11444 which was inserted into the XhoI and SspI sites of pOCUS-2. The fourth PCR fragment containing viral bases 10904 to 13773 was digested with PvuII and BamHI (viral bases 10918 to 13733) and inserted into the EcoRI (blunted with T4 DNA polymerase) and BamHI sites of pUC19. The two viral segments were combined by digesting the 7437/11444 clone with SacI (blunted with T4 DNA polymerase) and EcoNI and inserting into the 10918/13733 clone digested with EcoRJ (blunted with T4 DNA polymerase) and EcoNI. The resulting clone contained viral bases 7437 to 13733 in a pUC19 background. The remainder of the viral sequence was derived from a fifth PCR product encompassing viral bases 83 to 7457 which had been digested with XmnI and XhoI (viral bases 553 to 7437) and cloned into the StuI and XhoI sites of pOCUS-2. The 7437/13733 clone was then digested with BamHI, blunted with T4 DNA polymerase, and digested with XhoI to release a fragment that was inserted into the EagI (blunted with T4 DNA polymerase) and Xhol digested 553/7437 clone. The resulting clone contained viral bases 553 to 13733. This clone was then digested with PshAl and PacI and the resulting fragment containing viral bases 2143 to 13632 was inserted into the same sites of the pPIV3-MG(+) clone containing viral bases 1 to 2274 and 13632 to 15462. This generated pHPIV-3, the infectious clone, which is schematically depicted in Figure 4.

To insert the P gene into pGEM-4, P sequences were transferred from a P-lac-fusion clone, (described in 38, which is incorporated herein by reference) by digesting with XbaI (blunted with T4 DNA polymerase) BamHI, and inserted into the Kpnl (blunted with T4 DNA polymerase) and BamHI sites of pGEM4. The pPIV3-NP and pPIV3-L clones, as described in 15, 16, which are incorporated herein by reference., were in a pGEM-4 background. The pPIV3-L clone was also modified. In the natural L mRNA sequence there is a non-initiating AUG 11 nucleotides from the 5' end of the transcript. This was removed from pPIV3-L by mutational PCR, changing viral bases 8636 and 8637 from AT to TA.

### Example 2. Preparation of Recombinant HPIV-3.

Confluent monolayers of HeLa cells in 6-well plates were infected with recombinant vaccinia virus vTF7-3 at a multiplicity of infection of 2. vTF7-3 expresses T7 RNA polymerase as described in. Fuerst, T. R., P. L. Earl, and B. Moss. 1987. "Use of a hybrid vaccinia virus-T7 RNA polymerase system for expression of target genes." Mol. Cell. Biol. 7:2538-2544, and Fuerst, T. R., E. G. Niles, F. W. Studier, and B. Moss. 1986. "Eukaryotic transient-expression system based on recombinant vaccinia virus that synthesizes bacteriophage T7 RNA polymerase." Proc. Natl. Acad. Sci. 83:8122-8126, which are incorporated herein by reference. After 1 hour at 37°C, pPIV3-NP, pPIV3-P, pPIV3-L and pHPIV-3 were transfected using Lipofectin (BRL) according to manufacturers instructions. After three hours the transfection medium was removed and replaced with 1.5 ml Dulbecco's modified Eagle's medium (DMEM)/5% fetal bovine serum. After 40 to 48 hours the plates were frozen, thawed and scraped. The clarified medium supernatant (250 µl) was then used to infect fresh HeLa cell monolayers in 6-well plates. DMEM (1.5ml) containing 25 µg/ml 1-B-D-arabinofuranosylcytosine (araC) to inhibit vaccinia virus replication was added after a 1 hour attachment. After forty hours the plates were frozen, thawed, and scraped. The clarified medium supernatant was then titered for HPIV-3 in the presence of AraC. During the titering, isolated plaques were picked as agar plugs. The agar plugs were placed in 500 ml opti-MEM at 4°C for 4 hr. 250 µl were then used to infect fresh HeLa cell monolayers for amplification of the plaque isolates for 40 hr.

In a preferred embodiment transfection conditions were I µg pHPIV-3, 2 µg pPIV3-NP, 4 µg pPIV3-P and 0.1 µg pPIV3-L. Under these conditions approximately 1000 pfu per 6 X 10⁵ cells were obtained during the initial transfection and 10⁶ pfu per 6 x 10⁵ cells after the amplification.

When individual plasmids were omitted from the transfection step it was observed that the pHPIV-3, pPIV3-P and pPIV3-L plasmids were required for recovery of virus but, surprisingly, as shown in Table 1 below, pPIV3-NP was not.

**Table 1**

| Plasmids Transfected | | | | |
|---|---|---|---|---|
| HPIV-3 | NP | P | L | Virus Recovery |
| + | + | + | + | 14/15^{a} |
| - | + | + | + | 0/2 |
| + | - | + | + | 3/3^{b} |
| + | + | - | + | 0/3 |
| + | + | + | - | 0/2 |

| | | | | |
|---|---|---|---|---|
| Table 1. Recovery of HPIV-3 from pHPIV-3. HeLa cell monolayers were infected with vTF7-3 and transfected with the indicated plasmids. After 40 hr cells were lysed and supernatants added to fresh HeLa cell monolayers in the presence of araC to inhibit vTF7-3 replication. These monolayers were then lysed and the supernatants assayed for HPIV-3. The number of experiments for which HPIV-3 was recovered per attempts is displayed under virus recovery. ^{A}The single experiment that did not yield HPIV-3 used 0.1 ug of the P plasmid. ^{B}The omission of the NP plasmid resulted in 3 to 5 fold lower titers of HPIV-3. | | | | |

### Characterization of Recovered Virus.

In order to characterize the recovered virus and to purify HPIV-3 from vTF7-3, plaques of which are only slightly smaller than those of HPIV-3, isolated plaques suspected to be HPIV-3 were picked and amplified in HeLa cells. The plaque purified and amplified virus isolates and appropriate controls were then analyzed in neutralization assays. Virus (isolates #3 and #5) was preincubated (30 min on ice) with 5 ul rabbit pre-immune serum, 5 ul rabbit polyclonal anti-HPIV-3 antisera, or assayed in the presence of 25ug/ml araC The sera was incubated with virus on ice for 30 min prior to a standard plaque assay. To allow maximal plaque development, the plates were then incubated at 37°C for 66 hr prior to staining with crystal violet. The results of the assay indicated that the plaque purified virus was completely inhibited by the anti-HPIV-3 antisera, while vTF7-3 was not. In contrast, the HPIV-3 isolates were not inhibited by AraC, whereas the vTF7-3 virus was completely inhibited. Interestingly, of the eight recombinant HPIV-3 isolates, four had plaque sizes identical to the parental HPIV-3 stock while four were slightly larger. The plaque size of isolate #3 was slightly larger than isolate #5 and the wild type HPIV-3 virus.

To determine whether the NP coding sequence of pHPIV-3, which shares the same position as luciferase in pPIV3-MG(+), was being expressed from pHPIV-3, pHPIV-3 and pPIV3-NP were separately transfected into vTF7-3 infected HeLa cells and cell lists prepared after 48 hr. The lysates were then analyzed by Western blotting using an anit-HPIV-3RNP antisera. This antisera recognizes primarily NP and reacts poorly with P.

Specifically, extracts (equivalent to 6x10⁴ cells) were run on SDS-10% PAGE and transferred to nitrocellulose membranes. The primary antibody was a rabbit polyclonal anti-RNP antisera diluted 1:1000. The secondary antibody was 1:1000 dilution of a goat anti-rabbit antibody conjugated to horseradish peroxidase. Visualization was through chemiluminescence (ECL kit, Amersham). As shown by the Western blot, HPIV-3 RNP recognized NP from the pPIV3-NP and pHPIV-3 transfected cell extracts and from purified HPIV-3 RNP. No proteins were recognized in a mock-transfected HeLa extract. Thus, it appears that NP is expressed from pHPIV-3, presumably being translated from the T7-directed, antigenomic RNA transcript.

To determine whether the recovered, recombinant virus had specific mutations in its genome, RNA was extracted from wild type and plaque isolated viruses and used for RT-PCR analysis using primers flanking the substitution mutations. Viral RNA was isolated from approximately 2x10⁷ plaque forming units (pfu) of plaque purified virus isolates #3, 5, 7 and 9, or wt HPIV-3 strain 47885/. Reverse transcription was carried out using Superscript II reverse transcriptase (BMB) at 44°C for 1 hr using oligonucleotides which primed at viral base 23 or 15100. The PCR was carried out with Expand Long polymerase (BMB) using second primers which result in amplification of viral bases 23 to 303, or 15100 to 15440.

PCR products encompassing viral bases 1 to 324 and 15080 to 15462 were generated from the indicated isolates, digested with SacI and StuI, respectively, and analyzed on a 1.4% agarose gel.. PCR products of the expected sizes were generated in a RT-dependent manner, indicating that the PCR products were derived from RNA rather than contaminating plasmid DNA.

As shown on the agarose gel, the sizes of the 1 to 324 and 15080 to 15462 PCR products are increased by 21 and 22 base pairs, respectively, over the length of the viral specific regions due to the inclusion of restriction enzyme sites in the amplification primers. Digestion with SacI showed that the mutation at base 94 was not present in the wild-type virus but was present in the plaque isolated viruses, indicating they are of recombinant origin. Similarly, PCR product derived from the region encompassing viral base 15389 of wild type HPIV-3 was not cleaved by StuI. However, only four of the eight plaque isolated viruses contained the mutation which creates the StuI site. Direct sequencing of the PCR products confirmed these results.

### DISCUSSION

A full-length plasmid clone of the HPIV-3 genome, pHPIV-3 was constructed.. Upon transfection of pHPIV-3 and plasmids encoding the viral NP, P and L proteins into vTF7-3-infected HeLa cells, recombinant HPIV-3 bearing genetic markers was efficiently recovered. Several interesting features of this system were noted. First, the viral NP protein could be expressed from the infectious clone, and this expression obviated the need for an NP support plasmid. It is believed that the NP protein is synthesized directly from the primary anitgenomic transcript after it is capped by the vaccinia virus capping enzyme.

Second, two recombinant viruses with distinct genotypes and phenotypes were produced, probably due to recombination between the pHPIV-3 and pPIV3-L plasmids, although the possibility that the reversion arises during RNA replication cannot be excluded. pPIV3-L contains the entire L 3' UTR and part of the trailer region, extending to base 15437, an overlap of 48 base pairs beyond the Stul site. This is ample room for recombination between plasmids to occur readily in vaccinia virus infected cells

From these results, there appears to be selection in the HPIV-3 system. All the large plaque virus isolates had reverted to a wild-type sequence at base 15389, while retaining the change at base 94. Since A94G is the only known alteration in these viruses from the parental virus, it appears that The isolates which retained both mutations had a plaque size identical to that of the parental (wild type) virus, but when the 15389 mutations was lost, plaque size increased, indicating that the mutation at base 15389 was detrimental in the context of the A94G mutation. There was one other known change between pHPIV-3 and the support plasmids. A non-initiating AUG exists in the natural L protein message. Since this AUG is only 11 nucleotides from the 5' end of the L mRNA and in a poor translation initiation context, it may not cause much interference with L mRNA translation. However, in the support plasmid pPIV3-L this non-initiating AUG is much further from the 5' end of the transcript where it is more likely to be recognized by ribosomes. This AUG was removed from pPIV3-L by changing bases 8636 and 8637 from AT to TA, destroying a Sphl site and creating a NheI site. To investigate whether this change was present in the recombinant virus and could be responsible for the large plaque phenotype, RT-PCR analysis was done. PCR products encompassing this site and derived from both the wild type and the plaque isolated viruses retained a wild type sequence, indicating recombination had not occurred over this region and that these changes could not account for any variance in plaque size.

The finding that recombination may occur between transfected plasmids indicates that care must be taken when introducing mutations into the paramyxovirus or rhabdovirus infectious clone systems. Mutations introduced within the NP, P or L sequences preferably are carried by both the support plasmids and the infectious clone. Otherwise, resultant virus may not carry the desired mutation. The only possible exception to this is the HPIV-3 system, in which the HPIV-3 infectious clone expresses NP, negating the need for the NP support plasmid. Still, it is preferred that the HPIV-3 NP support plasmid be included in the system, since significantly greater yields of HPIV-3 were obtained when the support NP plasmid was included in the transfection.

An infectious clone for HPIV-3 is useful for understanding the molecular biology of HPIV-3 and for developing a vaccine for this important pathogen. The ability to generate specific mutations within HPIV-3 makes all aspects of HPIV-3 replication amenable to study. Any mutation, including those studied previously in other contexts, can now be examined with this system. The ability to introduce specific mutations also permits the possibility of revertant analysis, which could refine our understanding of protein-protein or protein-RNA interactions.

The infectious clone is also useful for identifying mutations which attenuate the virus. Such virus is useful for developing new vaccine strains of HPIV-3. In addition, mutations present in a current candidate vaccine strain of HPIV-3 can be inserted into pHPIV-3. Through identifying multiple deleterious mutations, it should be possible to engineer several mutations affecting various steps in the virus life cycle into a single HPIV-3 strain. Such a virus should be highly attenuated and not readily able to revert.

While the invention has been described to some degree of particularity, various adaptations and modifications can be made without departing from the scope of the invention as defined in the appended claims

## Claims

1. A recombinant HPIV clone comprising:
a) a nucleotide sequence encoding a positive sense, antigenome of human parainfluenza virus type 3(HPIV-3); and
b) an RNA polymerase promoter operatively linked to said nucleotide sequence.

2. The clone of claim 1 wherein said clone further comprises a ribozyme sequence downstream from said antigenome-encoding sequence.

3. The clone of claim 1 wherein the RNA polymerase promoter is the T7 RNA polymerase promoter.

4. The clone of claim 2 wherein the ribozyme is an antigenomic ribozyme.

5. The clone of claim 2 further comprising an RNA polymerase terminator downstream of the ribozyme sequence.

6. The clone of claim 1 wherein the nucleotide sequence encodes a modified anti-genome of HPIV-3.

7. The clone of claim 6 wherein the modified HPIV-3 antigenome-encoding sequence comprises a mutation selected from the group consisting of a substitution of one or more nucleotides, a deletion of from 3 to 12 nucleotides, and an addition of from 3 to 12 nucleotides.

8. A method for preparing recombinant human parainfluenza virus type 3 comprising;
a) providing a recombinant system which comprises:
i) an HPIV clone comprising a nucleotide sequence encoding a positive sense, antigenome of human parainfluenza virus type 3 ;
ii) a support clone comprising a nucleotide sequence encoding a human parainfluenza virus type 3 L protein; and
iii) a support clone comprising a nucleotide sequence encoding a human parainfluenza virus type 3 P protein;
wherein the nucleotide sequence encoding the P protein and L protein may be on the same support clone or on separate support clones;
b) introducing the recombinant system of step (a) into host cells;
c) culturing the host cells of step (b) for a time sufficient to permit transfection of the host cells and formation of recombinant human parainfluenza virus type 3; and
d) recovering the recombinant human parainfluenza virus type 3 from the culture of transfected host cells.

9. The method of claim 8 wherein the antigenome-encoding sequence of the HPIV-3 clone is operatively-linked to an RNA polymerase promoter;
wherein the P protein-encoding sequences of the P support clones is operatively-linked to an RNA polymerase promoter;
wherein the L protein-encoding sequences of the L support clones is operatively-linked to an RNA polymerase promoter; and
wherein the host cells comprise an RNA polymerase corresponding to the RNA polymerase promoter of said HPIV-3 clone and said support clones.

10. The method of claim 9, wherein the host cells are infected with a viral recombinant capable of expressing the RNA polymerase prior to or in combination with introduction of the recombinant system into the host cells.

11. A host cell for producing a recombinant human parainfluenza virus type 3, said host cell comprising:
a) an HPIV-3 clone comprising a nucleotide sequence encoding a positive sense, antigenome of human parainfluenza virus type 3;
b) a support clone comprising a nucleotide sequence encoding a human parainfluenza virus type 3 L protein; and
c) a support clone comprising a nucleotide sequence encoding a human parainfluenza virus type 3 P protein;
wherein the nucleotide sequence encoding the P protein and L protein may be on the same support clone or on separate support clones.

12. The host cell of claim 11 further comprising a support clone comprising a nucleotide sequence encoding a human parainfluenza virus type 3 NP protein.

13. A host cell for producing a recombinant human parainfluenza virus type 3, said host cell comprising:
a) an HPIV clone comprising a nucleotide sequence encoding a positive sense, antigenome of human parainfluenza virus type 3, wherein said antigenome sequence comprises a site specific mutation;
b) a support clone comprising a nucleotide sequence encoding a human parainfluenza virus type 3 L protein; and
c) a support clone comprising a nucleotide sequence encoding a human parainfluenza virus type 3 P protein;
wherein the nucleotide sequences encoding the P protein and the L protein are on the same support clone or on separate support clones.

14. The host cell of claim 12 wherein the HPIV-3 clone further comprises an RNA polymerase promoter operatively linked to the HPIV-3 antigenomic sequence and wherein each of the support clones comprises an RNA polymerase promoter operatively linked to the HPIV-3 protein-encoding sequence of said support.

15. A method of introducing a site-specific mutation into the genome of a recombinant human parainfluenza virus type 3, comprising the following steps:
a) preparing a clone comprising a nucleotide sequence encoding a human parainfluenza viral type 3 antigenome having a mutation at a specific site;
b) co-transfecting host cells with the clone of step (a), a support clone comprising a nucleotide sequence encoding an HPIV-3 L protein, and a support clone comprising a nucleotide sequence encoding an HPIV-3 P protein;
wherein the nucleotide sequence encoding the P protein and L protein may be on the same support clone or on separate support clones; and
c) culturing the transfected host cells for a time sufficient to allow formation of a recombinant human parainfluenza virus type 3.

16. The method of claim 15 further comprising the step of transfecting the host cells with a support clone comprising a nucleotide sequence encoding an HPIV-3 NP protein.

17. The method of claim 15 wherein the clone of step (a) is prepared using polymerase chain reaction techniques and a clone comprising a nucleotide sequence encoding a human parainfluenza virus type 3 antigenome as a template, wherein the antigenome-encoding nucleotide sequence of said template clone lacks the site- specific mutation.

18. The clone of claim 6, wherein the modified antigenome sequence has one or more mutations in a cis-acting element or a protein coding region.

19. The clone of claim 6, wherein the mutation is in the region encoding the P protein or in the region encoding the L protein.

20. A host cell for producing a recombinant human parainfluenza virus type 3, said host cell comprising:
a) an HPIV clone comprising a nucleotide sequence encoding a positive sense, antigenome of human parainfluenza virus type 3, wherein said antigenome sequence comprises a site specific mutation;
b) a support clone comprising a nucleotide sequence encoding a human parainfluenza virus type 3 L protein; and
c) a support clone comprising a nucleotide sequence encoding a human parainfluenza virus type 3 P protein;
wherein the nucleotide sequences encoding the P protein and the L protein are on the same support clone or on separate support clones; and
wherein the mutation is in the L protein coding region of the antigenome encoding sequence of the HPIV clone, and wherein the support clone which comprises the L protein encoding sequence has a corresponding mutation therein.

21. A host cell for producing a recombinant human parainfluenza virus type 3, said host cell comprising:
a) an HPIV-3 clone comprising a nucleotide sequence encoding a positive sense, antigenome of human parainfluenza virus type 3, wherein said antigenome sequence comprises a site specific mutation;
b) a support clone comprising a nucleotide sequence encoding a human parainfluenza virus type 3 L protein; and
c) a support clone comprising a nucleotide sequence encoding a human parainfluenza virus type 3 P protein;
wherein the nucleotide sequences encoding the P protein and the L protein are on the same support clone or on separate support clones; and
wherein the mutation is in the P protein coding region of the antigenome encoding sequence of the HPIV clone, and wherein the support clone which comprises the P protein encoding sequence has a corresponding mutation therein.

22. A host cell for producing a recombinant human parainfluenza virus type 3, said host cell comprising:
a) an HPIV-3 clone comprising a nucleotide sequence encoding a positive sense, antigenome of human parainfluenza virus type 3, wherein said antigenome sequence comprises a site specific mutation;
b) a support clone comprising a nucleotide sequence encoding a human parainfluenza virus type 3 L protein;
c) a support clone comprising a nucleotide sequence encoding a human parainfluenza virus type 3 P protein; and
d) a support clone comprising a nucleotide sequence encoding a human parainfluenza virus type 3 NP protein;
wherein the nucleotide sequences encoding the P protein, L protein, and NP protein are on the same support clone or on separate support clones and
wherein the mutation is in the NP protein coding region of the antigenome encoding sequence of the HPIV clone, and wherein the support clone which comprises the NP protein encoding sequence has a corresponding mutation therein.

## Patentansprüche

1. Rekombinanter HPIV-Klon, der umfasst:
a) eine Nucleotidsequenz, die ein positiv orientiertes Antigenom des humanen Parainfluenza-Virus Typ 3(HPIV-3) kodiert und
b) einen RNA-Polymerasepromoter, der an diese Nucleotidsequenz operativ gebunden ist.

2. Klon nach Anspruch 1, wobei dieser Klon weiter eine Ribozymsequenz stromabwärts von dieser das Antigenom kodierenden Sequenz umfasst.

3. Klon nach Anspruch 1, wobei der RNA-Polymerasepromoter der T7 RNA-Polymerasepromoter ist.

4. Klon nach Anspruch 2, wobei das Ribozym ein antigenomisches Ribozym ist.

5. Klon nach Anspruch 2, der weiter einen RNA-Polymeraseterminator stromabwärts der Ribozymsequenz umfasst.

6. Klon nach Anspruch 1, wobei die Nucleotidsequenz ein modifiziertes Antigenom des HPIV-3 kodiert.

7. Klon nach Anspruch 6, wobei die das modifizierte HPIV-3-Antigenom kodierende Sequenz eine Mutation umfasst, die aus der aus einer Substitution eines Nucleotids oder mehrerer Nucleotide, einer Deletion von 3 bis 12 Nucleotiden und einer Hinzufügung von 3 bis 12 Nucleotiden bestehenden Gruppe ausgewählt ist.

8. Verfahren zur Zubereitung eines rekombinanten humanen Parainfluenza-Virus Typ 3 umfassend:
a) Herstellen eines rekombinanten Systems, das umfasst:
i) einen HPIV-Klon, der eine ein positiv orientiertes Antigenom des humanen Parainfluenza-Virus Typ 3 kodierende Nucleotidsequenz umfasst,
ii) einen Trägerklon, der eine Nucleotidsequenz umfasst, die ein humanes Parainfluenza-Virus Typ 3 L-Protein kodiert, und
iii) einen Trägerklon, der eine Nucleotidsequenz umfasst, die ein humanes Parainfluenza-Virus Typ 3 P-Protein kodiert,
wobei die das P-Protein und das L-Protein kodierende Nucleotidsequenz sich auf demselben Trägerklon oder auf getrennten Trägerklonen befinden kann;
b) Einführen des rekombinanten Systems des Schritts (a) in Wirtszellen;
c) Züchten der Wirtszellen des Schritts (b) während eines Zeitraums, der ausreicht, um die Transfektion der Wirtszellen und die Bildung des rekombinanten humanen Parainfluenza-Virus Typ 3 zu ermöglichen, und
d) Rückgewinnung des rekombinanten humanen Parainfluenza-Virus Typ 3 aus der Kultur der transfizierten Wirtszellen.

9. Verfahren nach Anspruch 8, wobei die das Antigenom kodierende Sequenz des HPIV-3-Klons an einen RNA-Polymerasepromoter operativ gebunden ist,
wobei die das P-Protein kodierenden Sequenzen der P-Trägerklone an einen RNA-Polymerasepromoter operativ gebunden sind,
wobei die das L-Protein kodierenden Sequenzen der L-Trägerklone an einen RNA-Polymerasepromoter operativ gebunden sind, und
wobei die Wirtszellen eine RNA-Polymerase umfassen, die dem RNA-Polymerasepromoter dieses HPIV-3-Klons und dieser Trägerklonen entspricht.

10. Verfahren nach Anspruch 9, wobei die Wirtszellen mit einem viralen rekombinanten System infiziert werden, das fähig ist, die RNA-Polymerase vor oder in Kombination mit dem Einführen des rekombinanten Systems in die Wirtszellen zu exprimieren.

11. Wirtszelle zur Erzeugung eines rekombinanten humanen Parainfluenza-Virus Typ 3, wobei diese Wirtszelle umfasst:
a) einen HPIV-3-Klon, der eine Nucleotidsequenz umfasst, die ein positiv orientiertes Antigenom des humanen Parainfluenza-Virus Typ 3 kodiert,
b) einen Trägerklon, der eine Nucleotidsequenz umfasst, die ein humanes Parainfluenza-Virus Typ 3 L-Protein kodiert, und
c) einen Trägerklon, der eine Nucleotidsequenz umfasst, die ein humanes Parainfluenza-Virus Typ 3 P-Protein kodiert,
wobei die das P-Protein und das L-Protein kodierende Nucleotidsequenz sich auf demselben Trägerklon oder auf getrennten Trägerklonen befinden kann.

12. Wirtszelle nach Anspruch 11, die weiter einen Trägerklon umfasst, der eine ein humanes Parainfluenza-Virus Typ 3 NP-Protein kodierende Nucleotidsequenz aufweist.

13. Wirtszelle zur Erzeugung eines rekombinanten humanen Parainfluenza-Virus Typ 3, wobei diese Wirtszelle umfasst:
a) einen HPIV-Klon, der eine Nucleotidsequenz umfasst, die ein positiv orientiertes Antigenom des humanen Parainfluenza-Virus Typ 3 kodiert, wobei diese Antigenomsequenz eine stellenspezifische Mutation aufweist,
b) einen Trägerklon, der eine Nucleotidsequenz umfasst, die ein humanes Parainfluenza-Virus Typ 3 L-Protein kodiert, und
c) einen Trägerklon, der eine Nucleotidsequenz umfasst, die ein humanes Parainfluenza-Virus Typ 3 P-Protein kodiert,
wobei die das P-Protein und das L-Protein kodierenden Nucleotidsequenzen sich auf demselben Trägerklon oder auf getrennten Trägerklonen befinden.

14. Wirtszelle nach Anspruch 12, wobei der HPIV-3-Klon weiter einen RNA-Polymerasepromoter umfasst, der an die HPIV-3-antigenomische Sequenz operativ gebunden ist, und wobei jeder der Trägerklone einen RNA-Polymerasepromoter umfasst, der an die das HPIV-3-Protein kodierende Sequenz dieses Trägers operativ gebunden ist.

15. Verfahren zum Einführen einer stellenspezifischen Mutation in das Genom eines rekombinanten humanen Parainfluenza-Virus Typ 3, das die folgenden Schritte umfasst:
a) Herstellen eines Klons, der eine Nucleotidsequenz umfasst, die ein humanes Antigenom des Parainfluenza-viralen Typs 3 kodiert, das eine Mutation an einer spezifischen Stelle aufweist;
b) Co-Transfektion von Wirtszellen mit dem Klon des Schritts (a), einem Trägerklon, der eine ein HPIV-3 L-Protein kodierende Nucleotidsequenz umfasst, und einem Trägerklon, der eine ein HPIV-3 P-Protein kodierende Nucleotidsequenz umfasst,
wobei die das P-Protein und L-Protein kodierende Nucleotidsequenz auf demselben Trägerklon oder auf getrennten Trägerklonen sein kann, und
c) Züchten der transfizierten Wirtszellen während eines Zeitraums, der ausreicht, um die Bildung eines rekombinanten humanen Parainfluenza-Virus Typ 3 zu ermöglichen.

16. Verfahren nach Anspruch 15, das weiter den Schritt der Transfektion der Wirtszellen mit einem Trägerklon umfasst, der eine ein HPIV-3-NP-Protein kodierende Nucleotidsequenz aufweist.

17. Verfahren nach Anspruch 15, wobei der Klon des Schritts (a) hergestellt wird unter Anwendung von Polymerase-Kettenreaktions-Techniken und eines Klons, der eine Nucleotidsequenz umfasst, die ein humanes Parainfluenza-Virus Typ 3 Antigenom als Matrize kodiert, wobei der das Antigenom kodierenden Nucleotidsequenz dieses Matrizenklons die stellenspezifische Mutation fehlt.

18. Klon nach Anspruch 6, wobei die modifizierte Antigenomsequenz eine Mutation oder mehrere Mutationen in einem cis-wirkenden Element oder in einer Proteinkodierungsregion aufweist.

19. Klon nach Anspruch 6, wobei sich die Mutation in der das P-Protein kodierenden Region oder in der das L-Protein kodierenden Region befindet.

20. Wirtszelle zur Herstellung eines rekombinanten humanen Parainfluenza-Virus Typ 3, wobei diese Wirtszelle umfasst:
a) einen HPIV-Klon, der eine Nucleotidsequenz umfasst, die ein positiv orientiertes Antigenom des humanen Parainfluenza-Virus Typ 3 kodiert, wobei diese Antigenomsequenz eine stellenspezifische Mutation aufweist,
b) einen Trägerklon, der eine Nucleotidsequenz umfasst, die ein humanes Parainfluenza-Virus Typ 3 L-Protein kodiert, und
c) einen Trägerklon, der eine Nucleotidsequenz umfasst, die ein humanes Parainfluenza-Virus Typ 3 P-Protein kodiert,
wobei die das P-Protein und das L-Protein kodierenden Nucleotidsequenzen sich auf demselben Trägerklon oder auf getrennten Trägerklonen befinden und
wobei die Mutation sich in der L-Proteinkodierungsregion der das Antigenom kodierenden Sequenz des HPIV-Klons befindet und wobei der Trägerklon, der die das L-Protein kodierende Sequenz umfasst, eine entsprechende Mutation darin aufweist.

21. Wirtszelle zur Herstellung eines rekombinanten humanen Parainfluenza-Virus Typ 3, wobei diese Wirtszelle umfasst:
a) einen HPIV-3-Klon, der eine Nucleotidsequenz umfasst, die ein positiv orientiertes Antigenom des humanen Parainfluenza-Virus Typ 3 kodiert, wobei diese Antigenomsequenz eine stellenspezifische Mutation aufweist,
b) einen Trägerklon, der eine Nucleotidsequenz umfasst, die ein humanes Parainfluenza-Virus Typ 3 L-Protein kodiert, und
c) einen Trägerklon, der eine Nucleotidsequenz umfasst, die ein humanes Parainfluenza-Virus Typ 3 P-Protein kodiert,
wobei die das P-Protein und das L-Protein kodierenden Nucleotidsequenzen sich auf demselben Trägerklon oder auf getrennten Trägerklonen befinden, und
wobei die Mutation sich in der P-Proteinkodierungsregion der das Antigenom kodierenden Sequenz des HPIV-Klons befindet und wobei der Trägerklon, der die das P-Protein kodierende Sequenz umfasst, eine entsprechende Mutation darin aufweist.

22. Wirtszelle zur Herstellung eines rekombinanten humanen Parainfluenza-Virus Typ 3, wobei diese Wirtszelle umfasst:
a) einen HPIV-3-Klon, der eine Nucleotidsequenz umfasst, die ein positiv orientiertes Antigenom des humanen Parainfluenza-Virus Typ 3 kodiert, wobei diese Antigenomsequenz eine stellenspezifische Mutation aufweist,
b) einen Trägerklon, der eine Nucleotidsequenz umfasst, die ein humanes Parainfluenza-Virus Typ 3 L-Protein kodiert,
c) einen Trägerklon, der eine Nucleotidsequenz umfasst, die ein humanes Parainfluenza-Virus Typ 3 P-Protein kodiert, und
d) einen Trägerklon, der eine Nucleotidsequenz umfasst, die ein humanes Parainfluenza-Virus Typ 3 NP-Protein kodiert,
wobei die das P-Protein, L-Protein und NP-Protein kodierenden Nucleotidsequenzen sich auf demselben Trägerklon oder auf getrennten Trägerklonen befinden, und
wobei die Mutation sich in der NP-Proteinkodierungsregion der das Antigenom kodierenden Sequenz des HPIV-Klons befindet und wobei der Trägerklon, der die das NP-Protein kodierende Sequenz umfasst, eine entsprechende Mutation darin aufweist.

## Revendications

1. Clone HPIV recombinant comprenant :
a) une séquence de nucléotides codant un antigénome sens positif, du virus parainfluenza humain de type 3 (HPIV-3) ; et
b) un promoteur d'ARN polymérase fonctionnellement lié à ladite séquence de nucléotides.

2. Clone selon la revendication 1, dans lequel ledit clone comprend en outre une séquence de ribozyme en aval de ladite séquence codant l'antigénome.

3. Clone selon la revendication 1, dans lequel le promoteur de l'ARN polymérase est le promoteur de l'ARN polymérase T7.

4. Clone selon la revendication 2, dans lequel le ribozyme est un ribozyme antigénomique.

5. Clone selon la revendication 2, comprenant en outre un terminateur de l'ARN polymérase en aval de la séquence de ribozyme.

6. Clone selon la revendication 1, dans lequel la séquence de nucléotides code un antigénome modifié de HPIV-3.

7. Clone selon la revendication 6, dans lequel la séquence codant l'antigénome modifié de HPIV-3 comprend une mutation choisie parmi le groupe constitué d'une substitution d'un ou plusieurs nucléotide(s), une délétion de 3 à 12 nucléotides, et une addition de 3 à 12 nucléotides.

8. Procédé de préparation d'un virus parainfluenza humain recombinant de type 3 comprenant ;
a) la fourniture d'un système recombinant qui comprend :
i) un clone HPIV comprenant une séquence de nucléotides codant un antigénome sens positif du virus parainfluenza humain de type 3 ;
ii) un clone de support comprenant une séquence de nucléotides codant une protéine L du virus parainfluenza humain de type 3 ; et
iii) un clone de support comprenant une séquence de nucléotides codant une protéine P du virus parainfluenza humain de type 3 ;
dans lequel la séquence de nucléotides codant la protéine P et la protéine L peut se situer sur le même clone de support ou sur des clones de support séparés ;
b) l'introduction du système recombinant de l'étape (a) dans des cellules hôtes ;
c) la culture des cellules hôtes de l'étape (b) sur une durée suffisante pour permettre la transfection des cellules hôtes et la formation du virus de parainfluenza humain recombinant de type 3 ; et
d) la récupération du virus parainfluenza humain recombinant de type 3 de la culture des cellules hôtes transfectées.

9. Procédé selon la revendication 8, dans lequel la séquence codant l'antigénome du clone HPIV-3 est fonctionnellement liée à un promoteur de l'ARN polymérase ;
dans lequel les séquences codant la protéine P des clones de support P sont fonctionnellement liées à un promoteur de l'ARN polymérase ;
dans lequel les séquences codant la protéine L des clones de support L sont fonctionnellement liées à un promoteur de l'ARN polymérase ; et
dans lequel les cellules hôtes comprennent une ARN polymérase correspondant au promoteur de l'ARN polymérase dudit clone HPIV-3 et desdits clones de support.

10. Procédé selon la revendication 9, dans lequel les cellules hôtes sont infectées par un recombinant viral capable d'exprimer l'ARN polymérase avant ou en combinaison avec l'introduction du système recombinant dans les cellules hôtes.

11. Cellule hôte pour la production d'un virus parainfluenza humain recombinant de type 3, ladite cellule hôte comprenant :
a) un clone HPIV-3 comprenant une séquence de nucléotides codant un antigénome sens positif du virus parainfluenza humain de type 3 ;
b) un clone de support comprenant une séquence de nucléotides codant une protéine L du virus parainfluenza humain de type 3 ; et
c) un clone de support comprenant une séquence de nucléotides codant une protéine P du virus parainfluenza humain de type 3 ;
dans laquelle la séquence de nucléotides codant la protéine P et la protéine L peut se situer sur le même clone de support ou sur des clones de support séparés.

12. Cellule hôte selon la revendication 11, comprenant en outre un clone de support comprenant une séquence de nucléotides codant une protéine NP du virus parainfluenza humain de type 3.

13. Cellule hôte pour la production d'un virus parainfluenza humain recombinant de type 3, ladite cellule hôte comprenant :
a) un clone HPIV comprenant une séquence de nucléotides codant un antigénome sens positif du virus parainfluenza humain de type 3, dans lequel ladite séquence antigénomique comprend une mutation spécifique du site ;
b) un clone de support comprenant une séquence de nucléotides codant une protéine L du virus parainfluenza humain de type 3 ; et
c) un clone de support comprenant une séquence de nucléotides codant une protéine P du virus parainfluenza humain de type 3 ;
dans laquelle les séquences de nucléotides codant la protéine P et la protéine L se situent sur le même clone de support ou sur des clones de support séparés.

14. Cellule hôte selon la revendication 12, dans laquelle le clone HPIV-3 comprend en outre un promoteur d'ARN polymérase fonctionnellement lié à la séquence antigénomique HPIV-3 et dans laquelle chacun des clones de support comprend un promoteur d'ARN polymérase fonctionnellement lié à la séquence codant la protéine HPIV-3 dudit support.

15. Procédé d'introduction d'une mutation spécifique du site dans le génome d'un virus parainfluenza humain recombinant de type 3, comprenant les étapes suivantes :
a) la préparation d'un clone comprenant une séquence de nucléotides codant un antigénome viral parainfluenza humain de type 3 ayant une mutation au niveau d'un site spécifique ;
b) la cotransfection des cellules hôtes avec le clone de l'étape (a), un clone de support comprenant une séquence de nucléotides codant une protéine L HPIV-3, et un clone de support comprenant une séquence de nucléotides codant une protéine P HPIV-3 ;
dans lequel la séquence de nucléotides codant la protéine P et la protéine L peut se situer sur le même clone de support ou sur des clones de support séparés ; et
c) la culture des cellules hôtes transfectées sur une durée suffisante pour permettre la formation d'un virus parainfluenza humain recombinant de type 3.

16. Procédé selon la revendication 15, comprenant en outre l'étape de transfection des cellules hôtes avec un clone de support comprenant une séquence de nucléotides codant une protéine NP HPIV-3.

17. Procédé selon la revendication 15, dans lequel le clone de l'étape (a) est préparé en utilisant les techniques de réaction en chaîne de polymérase et un clone comprenant une séquence de nucléotides codant un antigénome du virus parainfluenza humain de type 3 comme modèle, dans lequel la séquence de nucléotides codant l'antigénome dudit clone modèle ne possède pas la mutation spécifique du site.

18. Clone selon la revendication 6, dans lequel la séquence antigénomique modifiée possède une ou plusieurs mutation(s) dans un élément à action de type *cis* ou une région codante de protéine.

19. Clone selon la revendication 6, dans lequel la mutation se situe dans la région codant la protéine P ou dans la région codant la protéine L.

20. Cellule hôte pour la production d'un virus parainfluenza humain recombinant de type 3, ladite cellule hôte comprenant :
a) un clone HPIV comprenant une séquence de nucléotides codant un antigénome sens positif de virus parainfluenza humain de type 3, dans lequel ladite séquence antigénomique comprend une mutation spécifique du site ;
b) un clone de support comprenant une séquence de nucléotides codant une protéine L de virus parainfluenza humain de type 3 ; et
c) un clone de support comprenant une séquence de nucléotides codant une protéine P de virus parainfluenza humain de type 3 ;
dans laquelle les séquences de nucléotides codant la protéine P et la protéine L se situent sur le même clone de support ou sur des clones de support séparés ; et
dans laquelle la mutation se situe dans la région codante de la protéine L de la séquence codant l'antigénome du clone HPIV, et dans laquelle le clone de support qui comprend la séquence codante de la protéine L a une mutation correspondante à l'intérieur.

21. Cellule hôte pour la production d'un virus parainfluenza humain recombinant de type 3, ladite cellule hôte comprenant :
a) un clone HPIV-3 comprenant une séquence de nucléotides codant un antigénome sens positif de virus parainfluenza humain de type 3, dans lequel ladite séquence antigénomique comprend une mutation spécifique du site ;
b) un clone de support comprenant une séquence de nucléotides codant une protéine L de virus parainfluenza humain de type 3 ; et
c) un clone de support comprenant une séquence de nucléotides codant une protéine P de virus parainfluenza humain de type 3 ;
dans laquelle les séquences de nucléotides codant la protéine P et la protéine L se situent sur le même clone de support ou sur des clones de support séparés ; et
dans laquelle la mutation se situe dans la région codante de la protéine P de la séquence codant l'antigénome du clone HPIV, et dans laquelle le clone de support qui comprend la séquence codante de la protéine P possède une mutation correspondante à l'intérieur.

22. Cellule hôte pour la production d'un virus parainfluenza humain recombinant de type 3, ladite cellule hôte comprenant :
a) un clone HPIV-3 comprenant une séquence de nucléotides codant un antigénome sens positif de virus parainfluenza humain de type 3, dans lequel ladite séquence antigénomique comprend une mutation spécifique du site ;
b) un clone de support comprenant une séquence de nucléotides codant une protéine L de virus parainfluenza humain de type 3 ;
c) un clone de support comprenant une séquence de nucléotides codant une protéine P de virus parainfluenza humain de type 3 ; et
d) un clone de support comprenant une séquence de nucléotides codant une protéine NP de virus parainfluenza humain de type 3 ;
dans laquelle les séquences de nucléotides codant la protéine P, la protéine L, et la protéine NP se situent sur le même clone de support ou sur des clones de support séparés et
dans laquelle la mutation se situe dans la région codante de la protéine NP de la séquence codant l'antigénome du clone HPIV, et dans laquelle le clone de support qui comprend la séquence codante de la protéine NP possède une mutation correspondante à l'intérieur.


### SEQUENCE LISTING as Annex.

(1) GENERAL INFORMATION:
   (i) APPLICANT: The Cleveland Clinic Foundation
   (ii) TITLE OF INVENTION: Infectious Clone for Human Parainfluenza Virus Type 3
   (iii) NUMBER OF SEQUENCES: 5
   (iv) CORRESPONDENCE ADDRESS:
      (A) ADDRESSEE: Wyeth Laboratories
      (B) STREET: Huntercombe Lane South
      (C) CITY: Taplow, Maidenhead
      (D) COUNTRY: UK
      (E) ZIP: SL6 0PH
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Walters, Philip B.W.
      (B) REGISTRATION NUMBER:
      (C) REFERENCE/DOCKET NUMBER: ACY-33557
   (ix) TELECOMMUNICATION INFORMATION:
      (A) TELEPHONE: +66 1628 413873
      (B) TELEFAX: +66 1628 799098
(2) INFORMATION FOR SEQ ID NO:1:
   (i) SEQUENCE CHARACTERISTICS:
      (A) LENGTH: 15462 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:1:
   ACCAAACAAG AGAAGAAACT TGTTCGGAAA TATAAATTTA AATTAAAATT AACTTAGGAT 60
   TAAAGACATT GACTAGAAGT CAAGAAAAGG TCAAGAAAAG GGAACTCTAT AATTTCAAAA 120
   ATGTTGAGCC TATTTGATAC ATTTAATGCA CGTAGGCAAG AAAACATAAC AAAATCAGCT 180
   GGTGGAGCTA TCATTCCTGG ACAGAAAAAT ACTGTCTCCA TATTTGCCCT TGGACCGACA 240
   ATAACTGATG ACAATGAGAA AATGACATTA GCTCTTCTAT TTCTATCTCA TTCACTAGAT 300
   AATGAGAAAC AACATGCACA AAGGGCAGGG TTCTTGGTGT CTTTATTGTC AATGGCTTAT 360
   GCCAATCCAG AGCTTTACCT GACAACAAAT GGAAGTAATG CAGATGTTAA ATATGTCATA 420
   TATATGATTG AGAAAGATCT AAAACGGCAA AAGTATGGAG GATTTGTGGT TAAGACGAGA 480
   GAGATGATAT ATGAAAAGAC AACTGAGTGG ATATTTGGAA GTGACCTGGA TTATGACCAG 540
   GAAACTATGC TGCAGAACGG CAGAAACAAT TCAACGATTG AAGATCTTGT TCACACATTT 600
   GGGTATCCAT CATGTTTAGG AGCTCTTATA ATACAGATCT GGATAGTTTT GGTCAAAGCC 660
   ATCACTAGCA TCTCAGGGTT AAGAAAAGGC TTTTTCACTC GATTAGAGGC TTTCAGACAA 720
   GATGGAACAG TGCAAGCAGG GCTGGTATTG AGCGGTGACA GCGTGGATCA GATTGGGTCA 780
   ATCATGCGGT CTCAACAGAG CTTGGTAACT CTTATGGTTG AGACATTAAT AACAATGAAT 840
   ACTAGCAGAA ATGACCTCAC AACCATAGAA AAGAATATAC AAATTGTTGG TAACTACATA 900
   AGAGATGCAG GTCTTGCTTC ATTCTTCAAT ACAATCAGGT ATGGAATTGA GACTAGAATG 960
   GCAGCTTTGA CTCTATCTAC TCTCAGACCA GATATCAATA GATTAAAAGC TCTGATGGAA 1020
   TTGTATTTAT CAAAGGGACC ACGCGCTCCT TTTATCTGTA TCCTCAGAGA TCCTATACAT 1080
   GGTGAGTTCG CACCAGGCAA CTATCCTGCC ATATGGAGTT ATGCAATGGG GGTGGCAGTT 1140
   GTACAAAACA GAGCCATGCA ACAGTATGTG ACGGGAAGAT CATATCTAGA TATTGATATG 1200
   TTCCAGCTGG GACAAGCAGT AGCACGTGAT GCTGAAGCTC AGATGAGCTC AACACTGGAA 1260
   GATGAACTTG GAGTGACACA CGAAGCCAAA GAAAGCTTGA AAAGACATAT AAGGAACATA 1320
   AACAGTTCAG AGACATTTTT CCACAAACCA ACAGGCGGAT CAGCCATAGA GATGGCAATA 1380
   GATGAAGAGC CAGAACAATT TGAACACAGA GCAGATCAAG AACAAGATGG AGAACCTCAA 1440
   TCATCTATAA TCCAATATGC TTGGGCAGAA GGAAACAGAA GTGATGATCG GACCGAGCAA 1500
   GCTACAGAAT CCGACAATAT CAAGACTGAA CAACAAAACA TCAGAGACAG ACTAAACAAG 1560
   AGACTCAACG ACAAGAAGAA ACAAGGCAGT CAACCATCCA CCAATCCCAC AAACAGAACG 1620
   AACCAGGACG AAATAGACGA TCTGTTCAAT GCATTTGGAA GCAACTAACT GAGTCAACAT 1680
   TTTGATCTAA ATCAATAATA AATAAGAAAA ACTTAGGATT AAAGAATCCT ATCATACCAG 1740
   AACATAGAGT GGTAAATTTA GAGTCTGCTT GCAACTCAAT CAATAGAGAG TTGATCGAAA 1800
   GCGATGCTAA AAACTATCAA ATCATGGATT CTTGGGAAGA GGAACCAAGA GATAAATCAA 1860
   CTAATATCTC CTCGGCCCTC AACATCATTG AATTCATACT CAGCACCGAC CCCCAAGAAG 1920
   ACCTATCGGA AAACGACACA ATCAACACAA GAACCCAGCA ACTCAGCGCC ACCATCTGTC 1980
   AACCAGAAAT CAAACCAACA GAAACAAGTG AAAAAGTTAG TGGATCAACT GACAAAAATA 2040
   GACAGTCTGG GTCATCACAC GAATGTACAA CAGAAGCAAA AGATAGAAAT ATTGATCAGG 2100
   AAACTGTACA GGGAGGATCT GGGAGAAGAA GCAGCTCAGA TAGTAGAGCT GAGACTGTGG 2160
   TCTCTGGAGG AATCTCTGGA AGCATCACAG ATTCTAAAAA TGGAACCCAA AACACGGAGA 2220
   ATATTGATCT CAATGAAATT AGAAAGATGG ATAAGGACTC TATTGAGAGG AAAATGCGAC 2280
   AATCTGCAGA TGTTCCAAGC GAGATATCAG GAAGTGATGT CATATTTACA ACAGAACAAA 2340
   GTAGAAACAG TGATCATGGA AGAAGCTTGG AACCTATCAG TACACCTGAT ACAAGATCAA 2400
   TGAGTGTTGT TACTGCTGCG ACACCAGATG ATGAAGAAGA AATACTAATG AAAAATAGTA 2460
   GGATGAAGAA AAGTTCTTCA ACACACCAAG AAGATGACAA AAGAATTAAA AAAGGGGGGG 2520
   GGGGAAAAGG GAAAGACTGG TTTAAGAAAT CAAGAGATAC TGACAACCAG ACATCAACAT 2580
   CAGATCACAA ACCCACATCA AAAGGGCAAA AGAAAATCTC AAAAACAACA ACCACCAACA 2640
   CCGACACAAA GGGGCAAACA GAAACACAGA CAGAATCATC AGAAACACAA TCCCCATCAT 2700
   GGAATCCCAT TATCGACAAC AACACTGACC GAACCGAACA GACAAGCACA ACCCCCCCAA 2760
   CAACAACTCC CAGATCAACT CGTACAAAAG AATCAATCCG AACAAACTCT GAATCCAAAC 2820
   CCAAGACACA AAAGACAATT GGAAAGGAAA GGAAGGATAC AGAAGAGAGC AATCGATTTA 2880
   CAGAGAGGGC AATTACTCTA TTGCAGAATC TTGGTGTAAT TCAATCTACA TCAAAACTAG 2940
   ATTTATATCA AGACAAACGA GTTGTATGTG TAGCAAATGT ACTAAACAAT GTAGATACTG 3000
   CATCAAAGAT AGACTTCCTA GCAGGATTAG TCATAGGGGT TTCAATGGAC AATGACACAA 3060
   AATTAATACA GATACAAAAT GAAATGTTAA ACCTCAAAGC AGATCTAAAG AGAATGGACG 3120
   AATCACATAG AAGATTGATA GAAAATCAAA GAGAACAACT GTCATTGATC ACATCGTTAA 3180
   TTTCAAATCT TAAAATTATG ACTGAGAGAG GAGGAAAGAA AGACCAAAAT GAATCCAATG 3240
   AGAGAGTATC TATGATCAAG ACAAAATTGA AAGAAGAAAA GATCAAGAAA ACCAGGTTTG 3300
   ACCCACTTAT GGAGGCACAA GGTATTGACA AGAATATACC TGATCTATAT CGACATGCAG 3360
   GAAATACGTT AGAGAACGAC GTACAAGTTA AATCAGAGAT ATTAAGTTCA TACAACGAGT 3420
   CAAATGCAAC AAGACTAATA CCCAGAAAAG TGAGCAGTAC AATGAGATCA CTAGTTGCAG 3480
   TCATCAACAA CAGCAATCTC CCACAAAGCA GAAAACAATC ATATATAAAC GAACTCAAAC 3540
   ATTGCAAAAG TGATGAAGAA GTATCTGAAT TGATGGACAT GTTCAATGAA GATGTTAACA 3600
   ATTGCTAAAG ATCAAATAAA AAAAACAACA CCGAATAAAT AGACAAGAAA CAACAGTAGA 3660
   TCAAAACCTA TCAACACACA CAAAATCAAG CAGAGTGAAA CAATAGACAT CAATCAATAT 3720
   ACAAATAAGA AAAATTTAGG ATTAAAGAAT AAATTAATCC TTGTCCAAAA TGAGTATAAC 3780
   TAACTCTGCA ATATACACAT TCCCGGAGTC ATCATTCTCT GAGAATGGTC ATATAGAACC 3840
   ATTACCACTC AAAGTCAATG AACAGAGAAA AGCAGTACCT CACATTAGAG TTGCCAAAAT 3900
   CGGAAATCCA CCAAAACATG GATCCCGGTA TTTGGATGTC TTCTTACTCG GCTTCTTCGA 3960
   GATGGAACGA ATCAAAGACA AATACGGGAG TGTGAATGAT CTTGACAGTG ACCCGGGTTA 4020
   CAAAGTTTGT GGCTCTGGAT CATTACCAAT CGGATTAGCC AAATACACTG GGAATGACCA 4080
   GGAATTATTA CAACTAAACT GGACATAGAA CAGTCAAAGC GAAAGAAATG ATTGTTTATA 4140
   CGGTACAAAA TATAAAACCA GAACTGTACC CATGGTCCAG TAGACTAAGA AAAGGAATGT 4200
   TGTTCGATGC CAACAAAGTT GCTCTTGCTC CTCAATGTCT TCCACTAGAT AGGAGCATAA 4260
   AATTCAGAGT AATCTTCGTT AATTGTACGG CAATTGGATC AATAACCTTG TTTAAAATTC 4320
   CCAAGTCAAT GGCATCACTA TCTCTACCCA GCACAATATC AATCAATCTG CAGGTACACA 4380
   TCAAAACAGG GGTTCAGACT GATTCTAAAG GGATAGTTCA AATTTTGGAT GAGAAGGGTG 4440
   AAAAATCACT GAATTTCATG GTCCATCTCG GATTGATCAA AAGAAAAGTA GGCAGAATGT 4500
   ACTCTGTCGA GTACTGTAAA CAGAAAATCG AGAAAATGAG ATTGATATTT TCTTTGGGAT 4560
   TAGTTGGAGG AATCAGTCTT CATGTCAATG CAACTGGATC TATATCAAAA ACACTAGCAA 4620
   GTCAGCTGGT ATTCAAAAGG GAGATTTGTT ATCCCTTAAT GGATCTAAAT CCACATCTCA 4680
   ATCTAGTTAT CTGGGCTTCA TCAGTAGAGA TTACAAGAGT GGATGCAATT TTCCAACCTT 4740
   CTTTACCTGG CGAGTTCAGA TACTATCCTA ACATTATTGC AAAAGGAGTT GGGAAAATCA 4800
   AACAATGGAA CTAGTAATCT CTATTTTGAT CTGGATATAT CTATTAAGCC AAAGCAAATA 4860
   AGAGATAATC AAAAACTTAG GACAAAAGAA GTCAATACCA ACAACTATTA GCAGCCACAC 4920
   TCGCTGGAAC AAGAAAGAAG GGATAAAAAA AGTTTAACAG AAGAAACAAA AACAAAAAGC 4980
   ACAGAACACC AGAACAACAA GATCAAAACA CCCAACCCAC TCAAAACGAA AATCTCAAAA 5040
   GAGATTGGCA ACACAACAAA CACTGAACAT CATGCCAACC TCAATACTGC TAATTATTAC 5100
   AACCATGATT ATGGCATCTT TCTGCCAAAT AGATATCACA AAACTACAGC ATGTAGGTGT 5160
   ATTGGTTAAC AGTCCCAAAG GGATGAAGAT ATCACAAAAC TTTGAAACAA GATATCTAAT 5220
   TTTGAGCCTC ATACCAAAAA TAGAAGATTC TAACTCTTGT GGTGACCAAC AGATCAAGCA 5280
   ATACAAGAGG TTATTGGATA GACTGATCAT TCCTTTATAT GATGGATTAA GATTACAGAA 5340
   GGATGTGATA GTGTCCAATC AAGAATCCAA TGAAAACACT GACCCCAGAA CAAAACGATT 5400
   CTTTGGAGGG GTAATTGGAA CTATTGCTCT GGGAGTGGCA ACCTCAGCAC AAATTACAGC 5460
   GGCAGTTGCT CTGGTTGAAG CCAAGCAGGC AAGATCAGAC ATTGAAAAAC TCAAGGAAGC 5520
   AATCAGGGAC ACAAACAAAG CAGTGCAGTC AGTCCAGAGC TCCATAGGAA ATTTGATAGT 5580
   AGCAATTAAA TCGGTCCAGG ATTATGTCAA CAAAGAAATC GTGCCATCAA TTGCGAGATT 5640
   AGGTTGTGAA GCAGCAGGAC TTCAGTTAGG AATTGCATTA ACACAGCATT ACTCAGAATT 5700
   AACAAACATA TTCGGTGATA ACATAGGATC ATTACAAGAA AAAGGGATAA AATTACAAGG 5760
   TATAGCATCA TTATACCGCA CAAATATCAC AGAGATATTC ACAACATCAA CAGTTGATAA 5820
   ATATGATATT TATGATCTAT TATTTACAGA ATCAATAAAG GTGAGAGTTA TAGATGTTGA 5880
   CTTGAATGAT TACTCAATCA CCCTCCAAGT CAGACTCCCT TTATTAACTA GACTGCTGAA 5940
   CACCCAGATT TACAGAGTAG ATTCCATATC ATATAACATC CAAAACAGAG AATGGTATAT 6000
   CCCTCTTCCC AGCCACATCA TGACAAAAGG GGCATTTCTA GGTGGAGCAG ATGTCAAAGA 6060
   ATGTATAGAA GCATTCAGCA GTTATATATG CCCTTCTGAT CCAGGATTTG TACTAAACCA 6120
   TGAAATGGAG AGCTGTTTAT CAGGAAACAT ATCCCAATGT CCAAGAACCG TGGTTAAATC 6180
   AGACATTGTT CCAAGATATG CATTTGTCAA TGGAGGAGTG GTTGCAAATT GTATAACAAC 6240
   CACATGTACA TGCAACGGTA TCGGTAATAG AATCAATCAA CCACCTGATC AAGGAGTAAA 6300
   AATTATAACA CATAAAGAAT GTAATACAAT AGGTATCAAC GGAATGCTGT TCAATACAAA 6360
   TAAAGAAGGA ACTCTTGCAT TTTACACACC AAATGATATA ACATTAAACA ATTCTGTTGC 6420
   ACTTGATCCA ATTGACATAT CAATCGAGCT CAATAAGGCC AAATCAGATC TAGAAGAGTC 6480
   AAAAGAATGG ATAAGAAGGT CAAATCAAAA ACTAGATTCC ATTGGAAATT GGCATCAATC 6540
   TAGCACCACA ATCATAATTG TTTTGATAAT GATAATTATA TTGTTTATAA TTAATGTAAC 6600
   GATAATTATA ATTGCAGTTA AGTATTACAG AATTCAAAAG AGAAATCGAG TGGATCAAAA 6660
   TGATAAACCA TATGTATTAA CAAACAAATG ACAGATCTAT AGATCATTAG ATATTAAAAT 6720
   TATAAAAAAC TTAGGAGTAA AGTTACGCAA TTCAACTCTA CTCATATAAT TGAGAAAGAA 6780
   CCCAACAGAC AAATCCAAAT CCGAGATGGA ATACTGGAAG CACACCAATC ACGGGAAAGA 6840
   TGCTGGTAAT GAGCTGGAAA CATCCATGGC TACTCATGGC AACAAGATCA CCAACAAGAT 6900
   AACATATATA TTATGGACAA TAATCCTGGT GTTATTATCA ATAGTCTTCA TCATAGTGCT 6960
   AATTAATTCC ATCAAAAGTG AAAAAGCCCA TGAATCATTG CTACAAGACG TAAACAATGA 7020
   GTTTATGGAA GTTACAGAAA AGATCCAAAT GGCATCGGAT AATATTAATG ATCTAATACA 7080
   GTCAGGAGTG AATACAAGGC TTCTTACAAT TCAGAGTCAT GTCCAGAATT ATATACCGAT 7140
   ATCATTGACA CAACAAATGT CGGATCTTAG GAAATTCATT AGTGAAATTA CAATTAGGAA 7200
   TGATAATCGA GAAGTGCCTC CACAAAGAAT AACACATGAT GCGGGCATAA AACCTTTAAA 7260
   TCCAGATGAT TTTTGGAGAT GCACGTCTGG TCTTCCATCT TTAATGAAAA CTCCAAAAAT 7320
   AAGGTTAATG CCGGGGCCGG GATTATTAGC TATGCCAACG ACTGTTGATG GCTGTGTTAG 7380
   AACTCCGTCC TTAGTTATTA ATGATCTGAT TTATGCTTAT ACCTCAAATC TAATTACTCG 7440
   AGGTTGCCAG GATATAGGAA AATCATATCA AGTATTACAG ATAGGGATAA TAACTGTAAA 7500
   CTCAGACTTG GTACCTGACT TAAATCCTAG GATCTCTCAT ACTTTCAACA TAAATGACAA 7560
   TAGAAAGTCA TGTTCTCTAG CACTCCTAAA CACAGATGTA TATCAACTGT GTTCGACTCC 7620
   CAAAGTTGAT GAAAGATCAG ATTATGCATC ATCAGGCATA GAAGATATTG TACTTGATAT 7680
   CGTCAATCAT GATGGTTCAA TCTCAACAAC AAGATTTAAG AACAATAATA TAAGTTTTGA 7740
   TCAACCATAT GCGGCATTAT ACCCATCTGT TGGACCAGGG ATATACTACA AAGGCAAAAT 7800
   AATATTTCTC GGGTATGGAG GTCTTGAACA TCCAATAAAT GAGAATGCAA TCTGCAACAC 7860
   AACTGGGTGT CCCGGGAAAA CGCAGAGAGA CTGCAATCAG GCATCTCATA GTCCTTGGTT 7920
   TTCAGACAGA AGGATGGTCA ACTCCATTAT TGTTGTTGAC AAGGGCTTAA ACTCAATTCC 7980
   AAAGCTGAAG GTATGGACGA TATCCATGAG ACAAAATTAC TGGGGGTCAG AAGGAAGGCT 8040
   ACTTCTACTA GGTAACAAGA TCTATATATA TACAAGATCT ACAAGTTGGC ATAGCAAGTT 8100
   ACAATTAGGA ATAATTGATA TTACTGATTA CAGTGATATA AGAATAAAAT GGACATGGCA 8160
   TAATGTGCTA TCAAGACCAG GAAACAATGA ATGTCCATGG GGACATTCAT GCCCAGATGG 8220
   ATGTATAACA GGAGTATATA CTGATGCATA TCCACTCAAT CCCACAGGGA GCATTGTGTC 8280
   ATCTGTCATA TTAGACTCGC AAAAATCGAG AGTAAACCCA GTCATAACTT ACTCAACATC 8340
   AACTGAAAGG GTAAACGAGC TGGCCATCCG AAACAAAACA CTCTCAGCTG GATATACAAC 8400
   AACGAGCTGC ATTACACACT ATAACAAAGG ATATTGTTTT CATATAGTAG AAATAAATCA 8460
   TAAAAGCTTA GACACATTCC AACCTATGTT GTTCAAAACA GAGATTCCAA AAAGCTGCAG 8520
   TTAATCATAA TTAACCATAA TATGTATTAA CCTATCTATA ATACAAGTAT ATGATAAGTA 8580
   ATCAGCAATC AGACAATAGA TAAAAGAGAA ATATAAAAAA CTTAGGAGCA AAGCATGCTC 8640
   GAAAAATGGA CACTGAATCT AACAATGGCA CTGTATCTGA CATACTCTAT CCTGAGTGTC 8700
   ACCTTAATTC TCCTATCGTT AAGGGTAAAA TAGCACAATT ACACACTATT ATGAGTCTAC 8760
   CACAGCCTTA CGATATGGAT GACGACTCAA TACTAGTTAT CACTAGACAG AAAATAAAAC 8820
   TCAATAAATT AGATAAAAGA CAACGATCTA TTAGAAGATT AAAATTAATA TTAACTGAGA 8880
   AAGTGAATGA CTTAGGAAAA TACACATTTA TTAGATATCC AGAAATGTCA AAAGAAATGT 8940
   TCAAATTACA TATACCTGGT ATTAACAGTA AAGTGACTGA ATTATTACTT AAAGCAGATA 9000
   GAACATATAG TCAAATGACT GATGGATTAA GAGATCTATG GATTAATGTG CTATCGAAAT 9060
   TAGCCTCAAA AAATGATGGA AGCAATTATG ATCTTAATGA AGAAATTAAT AATATATCAA 9120
   AAGTTCACAC AACCTATAAA TCAGATAAAT GGTATAATCC ATTCAAAACA TGGTTCACTA 9180
   TCAAGTATGA TATGAGAAGA TTGCAAAAAG CTCGAAATGA GGTCACTTTT AATATGGGGA 9240
   AAGATTATAA CTTGTTAGAA GACCAGAAGA ATTTCTTATT GATACATCCA GAATTGGTTT 9300
   TAATATTAGA TAAACAAAAC TATAATGGTT ATCTAATTAC TCCTGAATTA GTATTGCCGT 9360
   ATTGTGACGT AGTTGAAGGC CGATGGAATA TAAGTGCATG TGCTAAGTTA GATCCAAAAT 9420
   TACAATCTAT GTATCAGAAA GGCAATAATC TGTGGGAAGT GATAGATAAA TTGTTTCCAA 9480
   TTATGGGAGA AAAGACATTT GATGTGATAT CATTATTAGA ACCACTTGCA TTATCTCTAA 9540
   TTCAAACTCA TGATCCTGTT AAACAATTAA GGGGAGCTTT TTTAAATCAT GTGTTATCCG 9600
   AGATGGAATT GATATTTGAA TCTAGAGAAT CGATTAAAGA ATTTCTGAGT GTAGATTACA 9660
   TTGATAAAAT CTTAGATATA TTTAATAAAT CTACAATAGA TGAAATAGCA GAGATTTTCT 9720
   CTTTTTTTAG AACATTTGGG CATCCTCCAT TAGAGGCTAG TATTGCAGCA GAAAAAGTTA 9780
   GAAAATATAT GTATATTGGG AAACAATTAA AATTTGACAC TATTAATAAA TGTCATGCTA 9840
   TCTTCTGTAC AATAATAATT AACGGATATA GAGAAAGGCA TGGTGGACAG TGGCCTCCTG 9900
   TGACATTACC TGATCATGCA CACGAATTCA TCATAAATGC TTACGGTTCA AATTCTGCGA 9960
   TATCATATGA AAACGCTGTT GATTATTACC AGAGCTTTAT AGGAATAAAA TTTAATAAAT 10020
   TCATAGAACC TCAGTTAGAT GAAGATTTGA CAATTTATAT GAAAGATAAA GCATTGTCTC 10080
   CAAAAAAATC AAACTGGGAC ACAGTTTCTC CTGCATCTAA TTTACTGTAC CGTACTAACG 10140
   CATCCAACGA ATCACGAAGA TTAGTTGAAA AATTTATAGC AGATAGTAAA TTTGATCCTA 10200
   ATCAGATATT AGATTATGTA GAATCTGGGG ACTGGTTAGA TGATCCAGAA TTTAATATTT 10260
   CTTATAGTCT TAAAGAAAAA GAGATCAAAC AAGAAGGTAG ACTCTTTGCA AAAATGACAT 10320
   ATAAAATGAG AGCTACACAA GTTTTATCAG AGACACTACT TGCAAATAAT ATAGGGAAAT 10380
   TCTTTCAAGA AAATGGGATG GTAAAAGGAG AGATTGAATT ACTTAAGAGA TTAACAACCA 10440
   TATCAATATC AGGAGTTCCA CGGTATAATG AAGTATACAA TAATTCTAAA AGTCATACAG 10500
   ATGATCTTAA AACCTACAAT AAAATAAGTA ATCTCAATTT GTCTTCTAAT CAGAAATCAA 10560
   AGAAATTTGA ATTCAAGTCA ACGGATATTT ACAATGATGG ATACGAGACT GTGAGCTGTT 10620
   TTCTAACAAC AGATCTCAAA AAATACTGTC TTAATTGGAG ATATGAATCA ACAGCTCTAT 10680
   TTGGAGAAAC TTGCAACCAA ATATTTGGAT TAAATAAATT GTTTAATTGG TTACACCCTC 10740
   GTCTTGAAGG AAGTACAATC TATGTAGGTG ATCCCTATTG TCCTCCATCA GATAAGGAAC 10800
   ATATATCATT AGAGGATCAC CCTGATTCTG GATTTTATGT TCATAACCCA AGAGGGGGTA 10860
   TAGAAGGATT TTGTCAAAAA TTGTGGACAC TCATATCTAT AAGTGCAATA CATCTAGCAG 10920
   CTGTTAGAAT AGGCGTAAGG GTAACTGCAA TGGTTCAAGG AGATAATCAA GCTATAGCTG 10980
   TAACAACAAG AGTACCCAAC AATTATGACT ACAGAGTTAA GAAGGAGATA GTTTATAAAG 11040
   ATGTGGTGAG ATTTTTTGAT TCATTAAGAG AAGTAATGGA TGATCTAGGT CATGAACTTA 11100
   AATTAAATGA AACAATTATA AGTAGCAAGA TGTTCATATA TAGCAAAAGA ATATATTACG 11160
   ATGGGAGAAT TCTTCCCCAA GCTCTGAAAG CATTATCTAG ATGTGTCTTC TGGTCAGAGA 11220
   CAGTAATAGA CGAAACAAGA TCAGCATCTT CAAACTTGGC AACATCATTT GCAAAAGCAA 11280
   TTGAGAATGG TTATTCACCT GTTCTAGGAT ATGCATGCTC AATTTTTAAG AACATTCAAC 11340
   AACTATATAT TGCCCTTGGG ATGAATATCA ATCCAACTAT AACACAGAAT ATCAAAGATT 11400
   TATATTTTAG GAATCCAAAT TGGATGCAAT ATGCATCTTT AATACCTGCT AGTGTTGGGG 11460
   GATTCAATTA CATGGCCATG TCAAGATGTT TTGTAAGGAA TATTGGCGAT CCATCAGTTG 11520
   CCGCATTAGC TGATATTAAA AGATTTATTA AGGCGAACCT ATTAGACCGA AGTGTTCTTT 11580
   ATAGGATTAT GAATCAAGAA CCAGGTGAGT CATCTTTTTT GGACTGGGCT TCAGACCCAT 11640
   ATTCATGCAA TTTACCACAA TCTCAAAATA TAACCACTAT GATAAAAAAT ATAACAGCAA 11700
   GAAATGTATT ACAAGATTCA CCGAATCCAT TATTATCTGG ATTATTCACA AATACAATGA 11760
   TAGAAGAAGA TGAAGAATTA GCTGAGTTCT TGATGGACAG GAAGGTAATT CTCCCTAGAG 11820
   TTGCACATGA TATTCTAGAT AATTCTCTCA CAGGAATCAG AAATGCTATA GCTGGAATGT 11880
   TAGATACGAC AAAATCTCTA ATTCGGGTTG GCATAAATAG AGGAGGACTG ACATACAGTT 11940
   TGTTGAGGAA AATCAGTAAT TACGATCTAG TACAATATGA AACACTAAGT AGGACTTTGC 12000
   GACTAATTGT AAGCGATAAA ATCAGGTATG AAGATATGTG TTCGGTAGAC CTTGCTATAG 12060
   CATTGCGTCA AAAGATGTGG ATTCATTTAT CAGGAGGAAG GATGATAAGT GGACTTGAAA 12120
   CACCTGATCC ATTAGAATTA CTATCTGGGG TGATAATAAC AGGATCGGAA CATTGTAAAA 12180
   TATGTTATTC TTCAGATGGC ACAAACCCAT ATACTTGGAT GTATTTACCG GGTAATATTA 12240
   AAATAGGATC AGCAGAAACA GGTATATCAT CATTGAGAGT TCCTTATTTT GGATCAGTCA 12300
   CTGATGAGAG ATCTGAGGCA CAATTGGGAT ATATCAAGAA TCTTAGTAAA CCTGCAAAAG 12360
   CCGCAATAAG AATAGCAATG ATATATACAT GGGCATTTGG TAATGATGAG ATATCTTGGA 12420
   TGGAGGCCTC ACAAATAGCA CAAACACGTG CAAATTTTAC ACTAGATAGT CTCAAAATTC 12480
   TAACACCGGT AGCTACATCA ACAAATTTAT CACACAGATT AAAGGATACT GCAACCCAGA 12540
   TGAAGTTCTC CAGTACATCA TTGATTAGGG TCAGCAGATT CATAACAATG TCCAATGATA 12600
   ACATGTCTAT CAAGGAAGCT AATGAGACCA AAGATACCAA TCTTATTTAT CAACAAATAA 12660
   TGTTAACAGG ATTAAGTGTT TTCGAATATT TATTTAGATT AGAAGAAACC ACAGGACACA 12720
   ACCCCATAGT TATGCATCTG CACATAGAAG ATGAGTGTTG TATTAAAGAA AGTTTTAATG 12780
   ATGAGCATAT TAATCCAGAG TCTACATTAG AATTAATTAG GTACCCTGAA AGTAATGAAT 12840
   TTATTTATGA TAAAGACCCG CTCAAGGACG TGGACTTATC AAAACTTATG GTTATTAAAG 12900
   ATCATTCTTA CACAATTGAT ATGAATTATT GGGATGATAC TGACATCATA CATGCAATTT 12960
   CAATATGTAC TGCAATTACA ATAGCAGACA CTATGTCACA ATTAGATCGA GATAACTTAA 13020
   AAGAGATAAT AGTCATTGCA AATGATGATG ATATTAATAG CTTAATCACT GAATTTTTGA 13080
   CTCTTGATAT ACTTGTATTT CTTAAGACAT TTGGTGGATT ATTAGTAAAT CAATTTGCAT 13140
   ACACTCTTTA TAGTTTAAAA ACCGAAGGTA GGGACCTCAT TTGGGATTAT ATAATGAGAA 13200
   CACTGAGAGA TACTTCCCAT TCAATATTAA AAGTATTATC TAATGCATTA TCTCATCCTA 13260
   AAGTATTCAA GAGGTTCTGG GATTGTGGAG TCTTAAACCC TATTTATGGC CCTAATACTG 13320
   CTAGTCAAGA CCAGATAAAA CTTGCCCTCT CTATATGTGA ATATTCACTA GATCTATTTA 13380
   TGAGAGAATG GTTGAATGGT GTATCACTTG AAATATACAT TTGTGACAGC GATATGGAAG 13440
   TTGCGAATGA TAGGAAACAA GCCTTTATTT CTAGACACCT TTCATTTGTT TGTTGTTTAG 13500
   CAGAAATTGC ATCTTTTGGA CCTAACCTGT TAAACTTAAC ATACTTAGAG AGACTTGATC 13560
   TATTGAAACA ATATCTTGAA TTAAATATTA AAGACGACCC TACTCTTAAA TATGTACAAA 13620
   TATCTGGATT ATTAATTAAA TCGTTCCCAT CAACTGTAAC ATACGTAAGA AAGACTGCAA 13680
   TCAAATATTT AAGGATTCGT GGTATTAGTC CACCTGAGGT AATTGATGAT TGGGATCCGA 13740
   TAGAAGATGA AAATATGCTG GATAACATTG TCAAAACTAT AAATGATAAT TGTAATAAAG 13800
   ATAATAAAGG GAATAAAATT AACAATTTCT GGGGACTAGC GCTTAAGAAC TATCAGGTCC 13860
   TTAAAATCAG ATCTATAACA AGTGATTCTG ATAATAATGA TAGATCAGAT GCTAGTACCG 13920
   GTGGTTTGAC ACTTCCTCAA GGGGGGAATT ATCTATCACA TCAATTGAGA TTATTCGGAA 13980
   TCAACAGCAC TAGTTGTCTG AAAGCTCTTG AGTTATCACA AATTTTAATG AAGGAAGTTA 14040
   ATAAAGACCA GGACAGGCTC TTCCTAGGAG AAGGAGCAGG AGCTATGCTA GCATGTTATG 14100
   ATGCCACATT AGGACCTGCA ATTAATTATT ATAATTCCGG TTTGAATATA ACAGATGTAA 14160
   TTGGTCAACG AGAATTGAAA ATATTCCCTT CAGAGGTATC ATTAGTAGGT AAAAAATTAG 14220
   GAAATGTGAC ACAGATCCTT AATAGGGTAA AAGTACTGTT CAATGGAAAT CCAAATTCAA   14280
   CATGGATAGG AAATATGGAA TGCGAGACGT TAATATGGAG TGAATTGAAT GATAAGTCTA   14340
   TTGGATTGGT ACATTGTGAT ATGGAAGGAG CTATCGGTAA ATCAGAAGAA ACTGTTCTAC   14400
   ATGAACACTA TAGTGTTATA AGAATTACAT ACTTGATTGG GGATGATGAT GTTGTTTTAA   14460
   TTTCCAAAAT TATACCTACA ATCACTCCGA ATTGGTCTAG AATACTTTAT CTATATAAGT   14520
   TATATTGGAA AGATGTAAGT ATAATATCAC TTAAAACTTC TAATCCTGCA TCAACAGAAT   14580
   TATATCTAAT TTCAAAAGAT GCGTATTGTA CTATAATGGA ACCTAGTGAA GTTGTTTTAT   14640
   CAAAACTTAA AAGATTGTCA CTCTTGGAAG AAAATAATCT ATTAAAATGG ATCATTTTAT   14700
   CAAAGAAGAA AAATAATGAA TGGTTACATC ATGAAATCAA AGAAGGGGAA AGAGATTATG   14760
   GAGTTATGAG ACCATATCAT ATGGCATTAC AAATTTTTGG ATTTCAAATC AATTTAAATC   14820
   ATCTGGCGAA AGAATTTTTA TCAACTCCAG ATCTGACTAA TATCAACAAT ATAATCCAAA   14880
   GTTTTCAGAG AACAATCAAG GATGTTTTGT TTGAATGGAT TAATATAACT CATGATGGTA   14940
   AGAGACATAA ATTAGGCGGG AGATATAACA TATTCCCACT GAAAAATAAG GGGAAATTAA   15000
   GACTGCTATC GAGAAGACTA GTATTAAGTT GGATTTCATT GTCATTATCG ACTCGATTAC   15060
   TTACAGGTCG TTTTCCTGAT GAAAAATTTG AACATAGAGC ACAGACTGGA TATGTGTCAT   15120
   TACCTGATAC TGATTTAGAA TCATTAAAGT TATTGTCGAA AAACACCATT AAGAATTACA   15180
   GAGAGTGTAT AGGGTCAATA TCATATTGGT TTCTAACCAA AGAAGTTAAA ATACTTATGA   15240
   AATTGATTGG TGGTGCTAAA TTATTAGGAA TTCCCAGACA ATATAAGGAA CCCGAAGAAC   15300
   AGTTATTAGA AGACTACAAT CAACATGATG AATTTGATAT AGATTAAAAT ACAAATACAA   15360
   TAAAGATATA TCCTAACCTT TATCATTAAG CCTAAAGATA GACAAAAAGT AAGAAAAACA   15420
   TGTAATATAT ATATACCAAA CAGAGTTCTT CTCTTGTTTG GT                      15462
(2) INFORMATION FOR SEQ ID NO:2:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:2:
      TAGTCGGCCC TAATACGACT CACTATAGGA CCAAACAAGA GAAGAAACT 49
(2) INFORMATION FOR SEQ ID NO:3:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:3:
      GAAATTATAG AGCTCCCTTT TCT 23
(2) INFORMATION FOR SEQ ID NO:4:
   (i) SEQUENCE CHARACTERISTICS:
      (A) LENGTH: 95 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:4:
   TAAGGCCTAA AGATAGACAA AAACTAAGAA AAACATGTAA TATATATATA CCAAACAGAG 60
   TTCTTCTCTT GTTTGGTGGG TCGGCATGGC ATCTC 95
(2) INFORMATION FOR SEQ ID NO:5:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:5:
      CTGGGTACCT CCCTTAGCCA TCCGAGT 27